# EUROPEAN PATENT SPECIFICATION

(11) **EP 3 356 515 B1**
(45) Date of publication and mention of the grant of the patent: **05.06.2024**
(21) Application number: 16852436.1
(22) Date of filing: 28.09.2016
(51) Int. Cl.: C12N 5/073, G01N 33/50, G01N 33/88

(54) **CELL POTENCY ASSAY**
ZELLPOTENZTEST
TEST D'ACTIVITÉ BIOLOGIQUE CELLULAIRE

(30) Priority: 29.09.2015 US 201562234404 P
(43) Date of publication of application: 08.08.2018
(73) Proprietor: Celularity Inc., Florham Park, NJ 07932 (US)
(72) Inventor: RUSSOTTI, Gregory, Stirling, NJ 07980 (US); FULLER, Jaymes, North Brunswick, NJ 08902 (US); WIWI, Christopher, Basking Ridge, NJ 07920 (US)
(74) Representative: Mewburn Ellis LLP
(86) International application number: PCT/US2016/054058
(87) International publication number: WO 2017/058838

(56) References cited:
- WO-A1-2011/127117
- WO-A1-2012/092485
- WO-A1-2016/191449
- WO-A2-00/27424
- WO-A2-2008/100498
- US-A1- 2013 183 272
- US-B1- 6 376 541
- WEI LIU ET AL: "Human placenta-derived adherent cells induce tolerogenic immune responses", CLINICAL & TRANSLATIONAL IMMUNOLOGY, vol. 3, no. 5, 2 May 2014 (2014-05-02), page e14, XP055540521, DOI: 10.1038/cti.2014.5
- Wei Liu ET AL: "Human placenta-derived adherent cells induce tolerogenic immune responses - Supplementary Information", Clinical & Translational Immunology, 1 May 2014 (2014-05-01), pages 1-10, XP055540527, Australia DOI: 10.1038/cti.2014.5 Retrieved from the Internet: URL:https://www.ncbi.nlm.nih.gov/pmc/artic les/PMC4232071/bin/cti20145x1.pdf [retrieved on 2019-01-10]
- D CARNOVALE ET AL: "Aspirin dose dependently inhibits the interleukin-1[beta]-stimulated increase in inducible nitric oxide synthase, nitric oxide, and prostaglandin E2 production in rat ovarian dispersates cultured in vitro", FERTILITY AND STERILITY., vol. 75, no. 4, 1 April 2001 (2001-04-01), pages 778-784, XP55521849, USA ISSN: 0015-0282, DOI: 10.1016/S0015-0282(00)01784-2
- SANCHEZ T ET AL: "Induction by interleukin-1[beta] peptide of prostaglandin E2 formation via enhanced prostaglandin H synthase-2 expression in 3T6 fibroblasts", BIOCHEMICAL PHARMACOLOGY, ELSEVIER, US, vol. 56, no. 6, 1 January 1998 (1998-01-01), pages 759-761, XP002363830, ISSN: 0006-2952, DOI: 10.1016/S0006-2952(98)00056-2

## Description

This application claims priority benefit of U.S. Provisional Patent Application No. 62/234,404, filed September 29, 2015.

### 1. FIELD

Provided herein are methods for assessing cell potency.

### 2. BACKGROUND

Cell therapies represent a new and exciting treatment modality across a wide range of therapeutic indications. Accurate, reproducible, and relevant assays for assessing potency of cells used in cell therapies are important for quality control purposes, for example, ensuring stability and consistency of cell-based therapeutic products. WO2012/092485A1 relates to enhancement of placental stem cells potency using modulatory RNA molecules.

### 3. SUMMARY

The invention is defined in the appended claims.

Provided herein are methods for assessing cell potency, e.g., assessing the potency of cells in a preparation of cells (e.g., a preparation of cells that belongs to a lot of cells intended for therapeutic use). The methods provided herein utilize an increased cell culture time, e.g., recovery time, prior to assessing cell potency, as compared to standard cell potency assays used in the art. It has been determined that incorporating such an increased cell culture time results in increased assay reliability and decreased variability across cell preparations taken from same cell lot, as well as cell preparations comprising the same cell type but taken from different cell lots.

In a specific embodiment, provided herein but not part of the claimed invention, is a method for assessing cell potency, said method comprising the following steps (i) culturing a population of cells, e.g., a population of cells intended/suitable for use as a cell therapy, for a time period that minimizes variability in cell number once the cells have been cultured for the time period; and (ii) performing an assay to determine whether the cells produce a marker that acts as a surrogate for potency of the cells.

In another specific embodiment, provided herein but not part of the claimed invention, is a method for assessing cell potency, said method comprising the following steps (i) obtaining a population of cells, e.g., a population of cells intended/suitable for use as a cell therapy; (ii) culturing the cells for a time period that minimizes variability in cell number once the cells have been cultured for the time period; and (iii) performing an assay to determine whether the cells produce a marker that acts as a surrogate for potency of the cells.

In another specific embodiment, provided herein but not part of the claimed invention, is a method for assessing cell potency, said method comprising the following steps (i) culturing a population of cells, e.g., a population of cells intended/suitable for use as a cell therapy, for about 48 hours; and (ii) performing an assay to determine whether the cells produce a marker that acts as a surrogate for potency of the cells.

In another specific embodiment, provided herein but not part of the claimed invention, is a method for assessing cell potency, said method comprising the following steps (i) obtaining a population of cells, e.g., a population of cells intended/suitable for use as a cell therapy; (ii) culturing the cells for about 48 hours; and (iii) performing an assay to determine whether the cells produce a marker that acts as a surrogate for potency of the cells.

In another specific embodiment, provided herein but not part of the claimed invention, is a method for assessing cell potency, said method comprising the following steps (i) culturing a population of cells, e.g., a population of cells intended/suitable for use as a cell therapy, for about 48 hours; and (ii) performing an assay to determine whether the cells produce prostaglandin E2 (PGE2), wherein expression of PGE2 is a surrogate for potency of the cells.

In another specific embodiment, provided herein but not part of the claimed invention, is a method for assessing cell potency, said method comprising the following steps (i) obtaining a population of cells, e.g., a population of cells intended/suitable for use as a cell therapy; (ii) culturing the cells for about 48 hours; and (iii) performing an assay to determine whether the cells produce prostaglandin E2 (PGE2), wherein expression of PGE2 is a surrogate for potency of the cells.

In another specific embodiment, provided herein but not part of the claimed invention, is a method for assessing cell potency, said method comprising the following steps (i) culturing a population of cells, e.g., a population of cells intended/suitable for use as a cell therapy, for a time period that minimizes variability in cell number once the cells have been cultured for the time period; (ii) further culturing the cells in a medium that comprises one or more agents capable of inducing production of one or more genes in the cells, e.g., to cause increase production of one or more proteins by the cells; and (iii) performing an assay to determine whether the cells produce a marker that acts as a surrogate for potency of the cells.

In another specific embodiment, provided herein but not part of the claimed invention, is a method for assessing cell potency, said method comprising the following steps (i) obtaining a population of cells, e.g., a population of cells intended/suitable for use as a cell therapy; (ii) culturing the cells for a time period that minimizes variability in cell number once the cells have been cultured for the time period; (iii) further culturing the cells in a medium that comprises one or more agents capable of inducing production of one or more genes in the cells, e.g., to cause increase production of one or more proteins by the cells; and (iv) performing an assay to determine whether the cells produce a marker that acts as a surrogate for potency of the cells.

In another specific embodiment, provided herein is a method for assessing cell potency, said method comprising the following steps (i) culturing obtaining a population of cells, e.g., a population of cells intended/suitable for use as a cell therapy, for about 48 hours; (ii) further culturing the cells in a medium that comprises one or more agents capable of inducing production of one or more genes in the cells, e.g., to cause increase production of one or more proteins by the cells; and (iii) performing an assay to determine whether the cells produce a marker that acts as a surrogate for potency of the cells.

In another specific embodiment, provided herein is a method for assessing cell potency, said method comprising the following steps (i) obtaining a population of cells, e.g., a population of cells intended/suitable for use as a cell therapy; (ii) culturing the cells for about 48 hours; (iii) further culturing the cells in a medium that comprises one or more agents capable of inducing production of one or more genes in the cells, e.g., to cause increase production of one or more proteins by the cells; and (iv) performing an assay to determine whether the cells produce a marker that acts as a surrogate for potency of the cells.

In another specific embodiment, provided herein is a method for assessing cell potency, said method comprising the following steps (i) culturing a population of cells, e.g., a population of cells intended/suitable for use as a cell therapy, for about 48 hours; (ii) further culturing the cells in a medium that comprises an agent capable of inducing production of PGE by the cells; and (iii) performing an assay to determine whether the cells produce PGE2, wherein expression of PGE2 is a surrogate for potency of the cells. In a specific embodiment, said agent capable of inducing production of PGE2 by the cells is interleukin-1 (IL-1) beta.

In another specific embodiment, provided herein is a method for assessing cell potency, said method comprising the following steps (i) obtaining a population of cells, e.g., a population of cells intended/suitable for use as a cell therapy; (ii) culturing the cells for about 48 hours; (iii) further culturing the cells in a medium that comprises an agent capable of inducing production of PGE by the cells; and (iv) performing an assay to determine whether the cells produce PGE2, wherein expression of PGE2 is a surrogate for potency of the cells. In a specific embodiment, said agent capable of inducing production of PGE2 by the cells is interleukin-1 (IL-1) beta.

The methods for assessing for cell potency described herein can be carried using any cell type, e.g., any cell type intended/suitable for use as a therapeutic. Exemplary cell types that can be used in the methods described herein include, without limitation, mesenchymal stem cells, bone marrow-derived mesenchymal stem cells (BM-MSCs), tissue culture plastic-adherent CD34-, CD10+, CD105+, CD200+ placental stem cells, embryonic stem cells, embryonic germ cells, induced pluripotent stem cells, mesenchymal stem cells, bone marrow-derived mesenchymal stem cells, bone marrow-derived mesenchymal stromal cells, tissue plastic-adherent placental stem cells (PDACs), umbilical cord stem cells, amniotic fluid stem cells, amnion derived adherent cells (AMDACs) (see US Patent Application Publication No. 2010/0124569), osteogenic placental adherent cells (OPACs) (see US Patent Application Publication No. 2010/0047214), adipose stem cells, limbal stem cells, dental pulp stem cells, myoblasts, endothelial progenitor cells, neuronal stem cells, exfoliated teeth derived stem cells, hair follicle stem cells, dermal stem cells, parthenogenically derived stem cells, reprogrammed stem cells, amnion derived adherent cells, side population stem cells; and natural killer cells, e.g., the natural killer cells described in US Patent Nos. 8,263,065 and 8,926,964 or in International Patent Application Publication Nos. WO 2014/028453 and WO 2014/123879.

In a specific embodiment, the cells used in the methods described herein are placental cells, e.g., placental stem cells. In a a specific embodiment, the placental cells used in the methods described herein adhere to tissue culture plastic and are CD34⁻, CD10⁺, CD105⁺ and CD200⁺, as detectable by, e.g., flow cytometry. Further characteristics of placental cells that can be used in the methods provided herein are described in Section 5.1.

In certain embodiments, the cells used in the methods provided herein are from a lot of cells that has been previously prepared. In certain embodiments, when the cells used in the methods provided herein are from a lot of cells that has been previously prepared, said cells have been preserved, e.g., cryopreserved, prior to use in the method. In certain embodiments, when the cells used in the methods provided herein are from a lot of cells that has been previously prepared, said cells have been not been preserved, e.g., have not been cryopreserved, prior to use in the method.

In a specific embodiment, the time period used in the methods provided herein that minimizes variability in cell number is greater than 24 hours. In another specific embodiment, the time period used in the methods provided herein that minimizes variability in cell number is or is about 48 hours. In another specific embodiment, the time period used in the methods provided herein that minimizes variability in cell number is or is about 65 hours. In certain embodiments, the time period used in the methods provided herein that minimizes variability in cell number is about 30 hours, about 36 hours, about 42 hours, about 48 hours, about 54 hours, about 60 hours, or about 66 hours. In certain embodiments, the time period used in the methods provided herein that minimizes variability in cell number is between 30 hours to 36 hours, 36 hours to 42 hours, 42 hours to 48 hours, 48 hours to 54 hours, 54 hous to 60 hours, or 60 hours to 66 hours.

In certain embodiments, the marker that acts as a surrogate for potency of the cells in the population of cells correlates with the mechanism of action (MOA) of the cells for which potency is being assessed using a method described herein. In certain embodiments, the marker that acts as a surrogate for potency of the cells in the population of cells correlates with immunosuppressive activity of the cells. In a specific embodiment, expression of PGE2 is a surrogate for potency of the cells used in the methods described herein. In another specific embodiment expression of one of one more of the following proteins (and/or the genes that encode them) is/are a surrogate for potency of the cells used in the methods described herein: ANG, EGF, ENA-78, FGF2, Follistatin, G-CSF, GRO, HGF, IL-6, IL-8, Leptin, MCP-1, MCP-3, PDGFB, PLGF, Rantes, TGFB 1, Thrombopoietin, TIMP1, TIMP2, uPAR, VEGF, VEGFD, angiopoietin-1, angiopoietin-2, PECAM-1 (CD31; platelet endothelial cell adhesion molecule), laminin and/or fibronectin. In a specific embodiment, said cells are CD34⁻, CD10⁺, CD105⁺ and CD200⁺ placental stem cells (see Section 5.1).

Detection of a marker that acts as a surrogate for cell potency can be accomplished using any method known in the art. In a specific embodiment, markers that act as a surrogate for cell potency are detected by ELISA. In another specific embodiment, markers that act as a surrogate for cell potency are detected by use of a MultiplexBead Assay. In another specific embodiment, markers that act as a surrogate for cell potency are detected using an assay that measures gene expression, e.g., RT-PCR.

In certain embodiments, the method of detection of a surrogate for cell potency requires collection of the conditioned media from the cell culture, e.g., collection of the medium from the cell culture after the recovery time and/or collection of the medium from the cell culture after further culturing the cells in a medium that comprises one or more agents capable of inducing production of one or more genes in the cells.

In certain embodiments, the cells used in the methods described herein are adherent cells. Culturing of such cells in the methods described herein can be accomplished using, e.g., tissue culture plates, e.g., 6-, 24-, 48-, or 96-well tissue culture plates. In a specific embodiment, 48-well tissue culture plates are used to culture adherent cells in the methods described herein.

In certain embodiments, the cells used in the methods described herein are non-adherent cells. Culturing of such cells in the methods described herein can be accomplished, e.g., using tissue culture plates, e.g., 6-, 24-, 48-, or 96-well tissue culture plates, or by using tubes, flasks, and/or other containers known in the art for culturing cells in suspension.

In certain embodiments, determination of a time period that minimizes variability in cell number once the cells have been cultured for the time period is accomplished quantitatively. In a specific embodiment, determination of a time period that minimizes variability in cell number once the cells have been cultured for the time period is accomplished by counting the cells in the cell culture at a given time point and comparing the number of cells counted to the number of cells counted from a culture of the same cells at a different, e.g., earlier or later, time point. In another specific embodiment, determination of a time period that minimizes variability in cell number once the cells have been cultured for the time period is accomplished by visually inspecting the cells in the cell culture at a given time point, determining the percent confluence of the cell culture, and comparing the percent confluence of the cell culture to the percent confluence of cells visually inspected from a culture of the same cells at a different, e.g., earlier or later, time point. Methods for visually inspecting cell cultures in real time are known in the art. In a specific embodiment, a time period that minimizes variability in cell number is identified if decreased variability in the amount of cells counted or in the percent confluence is detected from cell preparations taken from same cell lot, and/or if decreased variability in the amount of cells counted or in the percent confluence is detected from cell preparations comprising the same cell type but taken from different cell lots is determined.

In certain embodiments, determination of a time period that minimizes variability in cell number once the cells have been cultured for the time period is accomplished qualitatively. In a specific embodiment, a time period that minimizes variability in cell number is identified if descreased variability in the amount of a surrogate marker, e.g., levels of PGE2, detected from cell preparations taken from same cell lot is determined, and/or if descreased variability in the amount of a surrogate marker, e.g., levels of PGE2, detected from cell preparations comprising the same cell type but taken from different cell lots is determined.

In certain embodiments, if it is determined that the cells used in the methods described herein produce a marker that acts as a surrogate for potency of the cells, then the original non-cultured population of cells is divided into a lot of cells suitable for administration to human subjects. In a specific embodiment, the lot is cryopreserved.

In certain embodiments, if it is determined that the cells used in the methods described herein produce a marker that acts as a surrogate for potency of the cells, then cells corresponding to the population of cells (e.g., other cells from the same lot of cells) are administered to a subject, e.g., a human.

### 3.1 Definitions

As used herein, the term "about," when referring to a stated numeric value, indicates a value within plus or minus 10% of the stated numeric value.

As used herein, the term "derived" means isolated from or otherwise purified. For example, placental derived adherent cells are isolated from placenta. The term "derived" encompasses cells that are cultured from cells isolated directly from a tissue, *e*.*g*., the placenta, and cells cultured or expanded from primary isolates.

As used herein, the term "isolated cell," *e*.*g*., "isolated placental cell," "isolated placental stem cell," and the like, means a cell that is substantially separated from other, different cells of the tissue, *e*.*g*., placenta, from which the stem cell is derived. A cell is "isolated" if at least 50%, 60%, 70%, 80%, 90%, 95%, or at least 99% of the cells, *e*.*g*., non-stem cells, with which the stem cell is naturally associated, or stem cells displaying a different marker profile, are removed from the stem cell, *e*.*g*., during collection and/or culture of the stem cell.

As used herein, the term "population of isolated cells" means a population of cells that is substantially separated from other cells of a tissue, *e*.*g*., placenta, from which the population of cells is derived.

As used herein, the term "placental cell" refers to a stem cell or progenitor cell that is isolated from a mammalian placenta, *e*.*g*., as described in Section 5.1, below, or cultured from cells isolated from a mammalian placenta, either as a primary isolate or a cultured cell, regardless of the number of passages after a primary culture. In certain embodiments, the term "placental cells," as used herein does not, however, refer to trophoblasts, cytotrophoblasts, syncitiotrophoblasts, angioblasts, hemangioblasts, embryonic germ cells, embryonic stem cells, cells obtained from an inner cell mass of a blastocyst, or cells obtained from a gonadal ridge of a late embryo, *e*.*g*., an embryonic germ cell.

As used herein, a placental cell is "positive" for a particular marker when that marker is detectable above background. Detection of a particular marker can, for example, be accomplished either by use of antibodies, or by oligonucleotide probes or primers based on the sequence of the gene or mRNA encoding the marker. For example, a placental cell is positive for, *e*.*g*., CD73 because CD73 is detectable on placental cells in an amount detectably greater than background (in comparison to, *e.g.*, an isotype control). A cell is also positive for a marker when that marker can be used to distinguish the cell from at least one other cell type, or can be used to select or isolate the cell when present or expressed by the cell. In the context of, *e.g.*, antibody-mediated detection, "positive," as an indication a particular cell surface marker is present, means that the marker is detectable using an antibody, *e*.*g*., a fluorescently-labeled antibody, specific for that marker; "positive" also refers to a cell exhibiting the marker in an amount that produces a signal, *e*.*g*., in a cytometer, that is detectably above background. For example, a cell is "CD200⁺" where the cell is detectably labeled with an antibody specific to CD200, and the signal from the antibody is detectably higher than that of a control (*e*.*g*., background or an isotype control). Conversely, "negative" in the same context means that the cell surface marker is not detectable using an antibody specific for that marker compared a control (*e*.*g*., background or an isotype control). For example, a cell is "CD34⁻" where the cell is not reproducibly detectably labeled with an antibody specific to CD34 to a greater degree than a control (*e*.*g*., background or an isotype control). Markers not detected, or not detectable, using antibodies are determined to be positive or negative in a similar manner, using an appropriate control. For example, a cell or population of cells can be determined to be OCT-4⁺ if the amount of OCT-4 RNA detected in RNA from the cell or population of cells is detectably greater than background as determined, *e.g.*, by a method of detecting RNA such as RT-PCR, slot blots, *etc.* Unless otherwise noted herein, cluster of differentiation ("CD") markers are detected using antibodies. In certain embodiments, OCT-4 is determined to be present, and a cell is "OCT-4⁺" if OCT-4 is detectable using RT-PCR.

As used herein, the terms "subject," "patient," and "individual" may be used interchangeably to refer to a mammal to whom a cell therapeutic is intended for administration. In a specific embodiment, a subject is a human.

### 4. BRIEF DESCRIPTION OF THE DRAWINGS

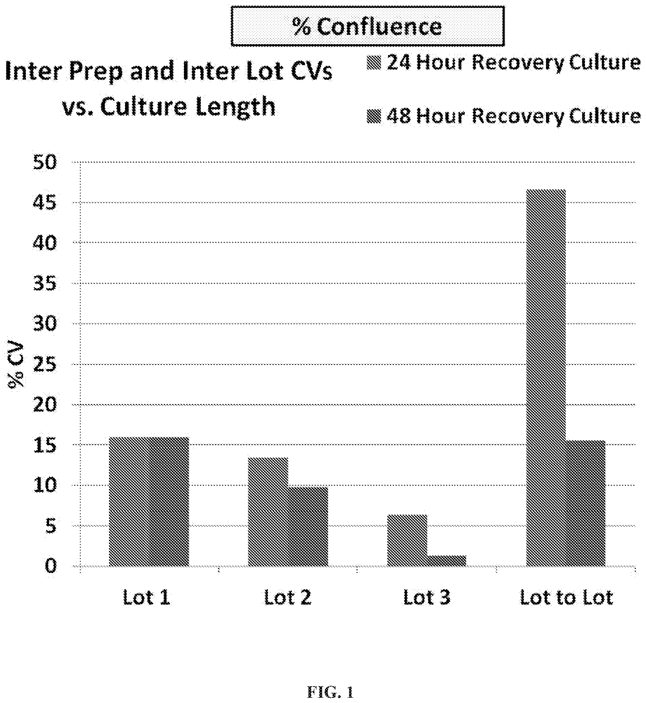

Figure 1 demonstrates that use of a 48 hour recovery culture period in a cell potency assay, as opposed to a 24-hour recovery culture period, results in improved inter-assay as well as intra-assay variability, as measured by percent confluence of the cells. 24-hour cultures: light gray shading; left bar above each lot number and above the "lot to lot" group. 48-hour cultures: dark gray shading; right bar above each lot number and above the "lot to lot" group.

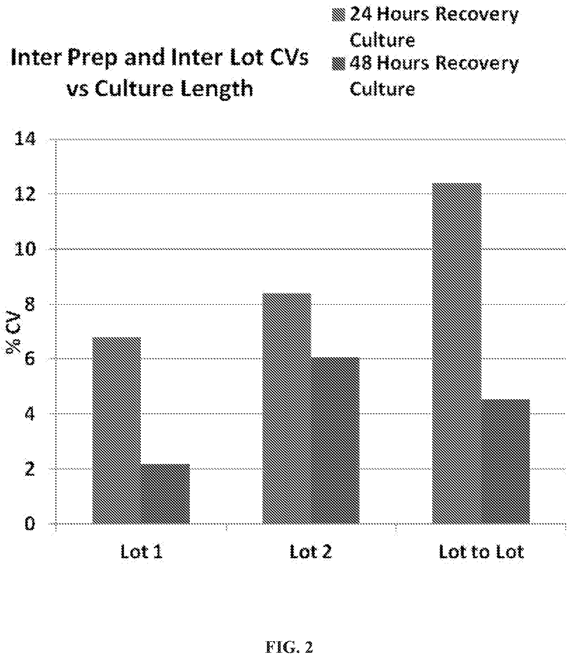

Figure 2 demonstrates that use of a 48 hour recovery culture period in a cell potency assay, as opposed to a 24-hour recovery culture period, results in improved inter-assay as well as intra-assay variability, as measured by percent confluence of the cells. 24-hour cultures: light gray shading; left bar above each lot number and above the "lot to lot" group. 48-hour cultures: dark gray shading; right bar above each lot number and above the "lot to lot" group.

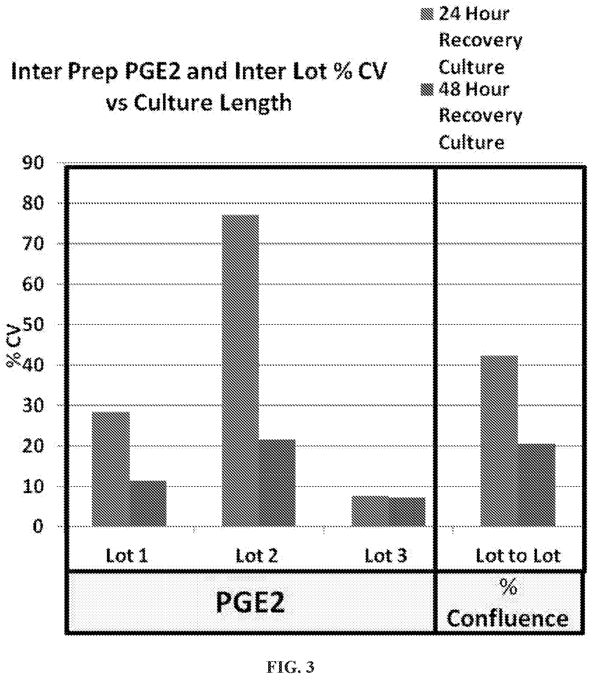

Figure 3 demonstrates that use of a 48 hour recovery culture period in a cell potency assay, as opposed to a 24-hour recovery culture period, results in improved variability as determined by measurement of PGE2 (left panel), and further confirms that use of a 48 hour recovery culture period in a cell potency assay, as opposed to a 24-hour recovery culture period, results in improved inter-assay variability, as measured by percent confluence of the cells (right panel). 24-hour cultures: light gray shading; left bar above each lot number and above the "lot to lot" group. 48-hour cultures: dark gray shading; right bar above each lot number and above the "lot to lot" group.

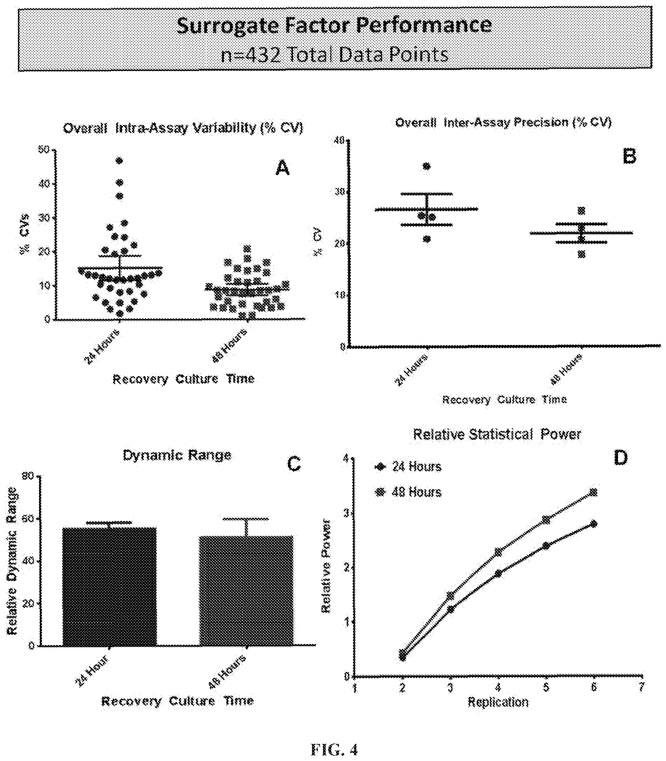

Figure 4 demonstrates that use of a 48 hour recovery culture period in a cell potency assay, as opposed to a 24-hour recovery culture period, results in reduced intra-assay variability (Figure 4A) and and reduced inter-assay variability (Figure 4B), does not affect dynamic range (Figure 4C), and results in increased statistical power (Figure 4D).

### 5. DETAILED DESCRIPTION

Provided herein are methods for assessing cell potency, e.g., assessing the potency of cells in a preparation of cells (e.g., a preparation of cells that belongs to a lot of cells intended for therapeutic use). The methods provided herein utilize an increased cell culture time, e.g., recovery time, prior to assessing cell potency, as compared to standard cell potency assays used in the art. It has been determined that incorporating such an increased cell culture time results in increased assay reliability and decreased variability across cell preparations taken from same cell lot, as well as cell preparations comprising the same cell type but taken from different cell lots.

In a specific embodiment, provided herein is a method for assessing cell potency, said method comprising the following steps (i) culturing a population of cells, e.g., a population of cells intended/suitable for use as a cell therapy, for a time period that minimizes variability in cell number once the cells have been cultured for the time period; and (ii) performing an assay to determine whether the cells produce a marker that acts as a surrogate for potency of the cells.

In another specific embodiment, provided herein is a method for assessing cell potency, said method comprising the following steps (i) obtaining a population of cells, e.g., a population of cells intended/suitable for use as a cell therapy; (ii) culturing the cells for a time period that minimizes variability in cell number once the cells have been cultured for the time period; and (iii) performing an assay to determine whether the cells produce a marker that acts as a surrogate for potency of the cells.

In another specific embodiment, provided herein is a method for assessing cell potency, said method comprising the following steps (i) culturing a population of cells, e.g., a population of cells intended/suitable for use as a cell therapy, for about 48 hours; and (ii) performing an assay to determine whether the cells produce a marker that acts as a surrogate for potency of the cells.

In another specific embodiment, provided herein is a method for assessing cell potency, said method comprising the following steps (i) obtaining a population of cells, e.g., a population of cells intended/suitable for use as a cell therapy; (ii) culturing the cells for about 48 hours; and (iii) performing an assay to determine whether the cells produce a marker that acts as a surrogate for potency of the cells.

In another specific embodiment, provided herein is a method for assessing cell potency, said method comprising the following steps (i) culturing a population of cells, e.g., a population of cells intended/suitable for use as a cell therapy, for about 48 hours; and (ii) performing an assay to determine whether the cells produce prostaglandin E2 (PGE2), wherein expression of PGE2 is a surrogate for potency of the cells.

In another specific embodiment, provided herein is a method for assessing cell potency, said method comprising the following steps (i) obtaining a population of cells, e.g., a population of cells intended/suitable for use as a cell therapy; (ii) culturing the cells for about 48 hours; and (iii) performing an assay to determine whether the cells produce prostaglandin E2 (PGE2), wherein expression of PGE2 is a surrogate for potency of the cells.

In another specific embodiment, provided herein is a method for assessing cell potency, said method comprising the following steps (i) culturing a population of cells, e.g., a population of cells intended/suitable for use as a cell therapy, for a time period that minimizes variability in cell number once the cells have been cultured for the time period; (ii) further culturing the cells in a medium that comprises one or more agents capable of inducing production of one or more genes in the cells, e.g., to cause increase production of one or more proteins by the cells; and (iii) performing an assay to determine whether the cells produce a marker that acts as a surrogate for potency of the cells.

In another specific embodiment, provided herein is a method for assessing cell potency, said method comprising the following steps (i) obtaining a population of cells, e.g., a population of cells intended/suitable for use as a cell therapy; (ii) culturing the cells for a time period that minimizes variability in cell number once the cells have been cultured for the time period; (iii) further culturing the cells in a medium that comprises one or more agents capable of inducing production of one or more genes in the cells, e.g., to cause increase production of one or more proteins by the cells; and (iv) performing an assay to determine whether the cells produce a marker that acts as a surrogate for potency of the cells.

In another specific embodiment, provided herein is a method for assessing cell potency, said method comprising the following steps (i) culturing obtaining a population of cells, e.g., a population of cells intended/suitable for use as a cell therapy, for about 48 hours; (ii) further culturing the cells in a medium that comprises one or more agents capable of inducing production of one or more genes in the cells, e.g., to cause increase production of one or more proteins by the cells; and (iii) performing an assay to determine whether the cells produce a marker that acts as a surrogate for potency of the cells.

In another specific embodiment, provided herein is a method for assessing cell potency, said method comprising the following steps (i) obtaining a population of cells, e.g., a population of cells intended/suitable for use as a cell therapy; (ii) culturing the cells for about 48 hours; (iii) further culturing the cells in a medium that comprises one or more agents capable of inducing production of one or more genes in the cells, e.g., to cause increase production of one or more proteins by the cells; and (iv) performing an assay to determine whether the cells produce a marker that acts as a surrogate for potency of the cells.

In another specific embodiment, provided herein is a method for assessing cell potency, said method comprising the following steps (i) culturing a population of cells, e.g., a population of cells intended/suitable for use as a cell therapy, for about 48 hours; (ii) further culturing the cells in a medium that comprises an agent capable of inducing production of PGE by the cells; and (iii) performing an assay to determine whether the cells produce PGE2, wherein expression of PGE2 is a surrogate for potency of the cells. In a specific embodiment, said agent capable of inducing production of PGE2 by the cells is interleukin-1 (IL-1) beta.

In another specific embodiment, provided herein is a method for assessing cell potency, said method comprising the following steps (i) obtaining a population of cells, e.g., a population of cells intended/suitable for use as a cell therapy; (ii) culturing the cells for about 48 hours; (iii) further culturing the cells in a medium that comprises an agent capable of inducing production of PGE by the cells; and (iv) performing an assay to determine whether the cells produce PGE2, wherein expression of PGE2 is a surrogate for potency of the cells. In a specific embodiment, said agent capable of inducing production of PGE2 by the cells is interleukin-1 (IL-1) beta.

The methods for assessing for cell potency described herein can be carried using any cell type, e.g., any cell type intended/suitable for use as a therapeutic. Exemplary cell types that can be used in the methods described herein include, without limitation, mesenchymal stem cells, bone marrow-derived mesenchymal stem cells (BM-MSCs), tissue culture plastic-adherent CD34-, CD10+, CD105+, CD200+ placental stem cells, embryonic stem cells, embryonic germ cells, induced pluripotent stem cells, mesenchymal stem cells, bone marrow-derived mesenchymal stem cells, bone marrow-derived mesenchymal stromal cells, tissue plastic-adherent placental stem cells (PDACs), umbilical cord stem cells, amniotic fluid stem cells, amnion derived adherent cells (AMDACs) (see US Patent Application Publication No. 2010/0124569), osteogenic placental adherent cells (OPACs) (see US Patent Application Publication No. 2010/0047214), adipose stem cells, limbal stem cells, dental pulp stem cells, myoblasts, endothelial progenitor cells, neuronal stem cells, exfoliated teeth derived stem cells, hair follicle stem cells, dermal stem cells, parthenogenically derived stem cells, reprogrammed stem cells, amnion derived adherent cells, side population stem cells; and natural killer cells, e.g., the natural killer cells described in US Patent Nos. 8,263,065 and 8,926,964 or in International Patent Application Publication Nos. WO 2014/028453 and WO 2014/123879.

In a specific embodiment, the cells used in the methods described herein are placental cells, e.g., placental stem cells. In a a specific embodiment, the placental cells used in the methods described herein adhere to tissue culture plastic and are CD34⁻, CD10⁺, CD105⁺ and CD200⁺, as detectable by, e.g., flow cytometry. Further characteristics of placental cells that can be used in the methods provided herein are described in Section 5.1.

In certain embodiments, the cells used in the methods provided herein are from a lot of cells that has been previously prepared. In certain embodiments, when the cells used in the methods provided herein are from a lot of cells that has been previously prepared, said cells have been preserved, e.g., cryopreserved, prior to use in the method. In certain embodiments, when the cells used in the methods provided herein are from a lot of cells that has been previously prepared, said cells have been not been preserved, e.g., have not been cryopreserved, prior to use in the method.

In a specific embodiment, the time period used in the methods provided herein that minimizes variability in cell number is greater than 24 hours. In another specific embodiment, the time period used in the methods provided herein that minimizes variability in cell number is or is about 48 hours. In another specific embodiment, the time period used in the methods provided herein that minimizes variability in cell number is or is about 65 hours. In certain embodiments, the time period used in the methods provided herein that minimizes variability in cell number is about 30 hours, about 36 hours, about 42 hours, about 48 hours, about 54 hours, about 60 hours, or about 66 hours. In certain embodiments, the time period used in the methods provided herein that minimizes variability in cell number is between 30 hours to 36 hours, 36 hours to 42 hours, 42 hours to 48 hours, 48 hours to 54 hours, 54 hous to 60 hours, or 60 hours to 66 hours.

In certain embodiments, the marker that acts as a surrogate for potency of the cells in the population of cells correlates with the mechanism of action (MOA) of the cells for which potency is being assessed using a method described herein. In certain embodiments, the marker that acts as a surrogate for potency of the cells in the population of cells correlates with immunosuppressive activity of the cells. In a specific embodiment, expression of PGE2 is a surrogate for potency of the cells used in the methods described herein. In another specific embodiment expression of one of one more of the following proteins (and/or the genes that encode them) is/are a surrogate for potency of the cells used in the methods described herein: ANG, EGF, ENA-78, FGF2, Follistatin, G-CSF, GRO, HGF, IL-6, IL-8, Leptin, MCP-1, MCP-3, PDGFB, PLGF, Rantes, TGFB 1, Thrombopoietin, TIMP1, TIMP2, uPAR, VEGF, VEGFD, angiopoietin-1, angiopoietin-2, PECAM-1 (CD31; platelet endothelial cell adhesion molecule), laminin and/or fibronectin. In a specific embodiment, said cells are CD34⁻, CD10⁺, CD105⁺ and CD200⁺ placental stem cells (see Section 5.1).

Detection of a marker that acts as a surrogate for cell potency can be accomplished using any method known in the art. In a specific embodiment, markers that act as a surrogate for cell potency are detected by ELISA. In another specific embodiment, markers that act as a surrogate for cell potency are detected by use of a MultiplexBead Assay. In another specific embodiment, markers that act as a surrogate for cell potency are detected using an assay that measures gene expression, e.g., RT-PCR.

In certain embodiments, the method of detection of a surrogate for cell potency requires collection of the conditioned media from the cell culture, e.g., collection of the medium from the cell culture after the recovery time and/or collection of the medium from the cell culture after further culturing the cells in a medium that comprises one or more agents capable of inducing production of one or more genes in the cells.

In certain embodiments, the cells used in the methods described herein are adherent cells. Culturing of such cells in the methods described herein can be accomplished using, e.g., tissue culture plates, e.g., 6-, 24-, 48-, or 96-well tissue culture plates. In a specific embodiment, 48-well tissue culture plates are used to culture adherent cells in the methods described herein.

In certain embodiments, the cells used in the methods described herein are non-adherent cells. Culturing of such cells in the methods described herein can be accomplished, e.g., using tissue culture plates, e.g., 6-, 24-, 48-, or 96-well tissue culture plates, or by using tubes, flasks, and/or other containers known in the art for culturing cells in suspension.

In certain embodiments, determination of a time period that minimizes variability in cell number once the cells have been cultured for the time period is accomplished quantitatively. In a specific embodiment, determination of a time period that minimizes variability in cell number once the cells have been cultured for the time period is accomplished by counting the cells in the cell culture at a given time point and comparing the number of cells counted to the number of cells counted from a culture of the same cells at a different, e.g., earlier or later, time point. In another specific embodiment, determination of a time period that minimizes variability in cell number once the cells have been cultured for the time period is accomplished by visually inspecting the cells in the cell culture at a given time point, determining the percent confluence of the cell culture, and comparing the percent confluence of the cell culture to the percent confluence of cells visually inspected from a culture of the same cells at a different, e.g., earlier or later, time point. Methods for visually inspecting cell cultures in real time are known in the art. In a specific embodiment, a time period that minimizes variability in cell number is identified if decreased variability in the amount of cells counted or in the percent confluence is detected from cell preparations taken from same cell lot, and/or if decreased variability in the amount of cells counted or in the percent confluence is detected from cell preparations comprising the same cell type but taken from different cell lots is determined.

In certain embodiments, determination of a time period that minimizes variability in cell number once the cells have been cultured for the time period is accomplished qualitatively. In a specific embodiment, a time period that minimizes variability in cell number is identified if descreased variability in the amount of a surrogate marker, e.g., levels of PGE2, detected from cell preparations taken from same cell lot is determined, and/or if descreased variability in the amount of a surrogate marker, e.g., levels of PGE2, detected from cell preparations comprising the same cell type but taken from different cell lots is determined.

### 5.1 ISOLATED PLACENTAL CELLS AND ISOLATED PLACENTAL CELL POPULATIONS

The isolated placental cells, sometimes referred to herein as PDACs (and also sometimes designated "PDA-002"), useful in the methods provided herein are obtainable from a placenta or part thereof, adhere to a tissue culture substrate and have characteristics of multipotent cells or stem cells, but are not trophoblasts. In certain embodiments, the isolated placental cells useful in the methods disclosed herein have the capacity to differentiate into non-placental cell types.

The isolated placental cells useful in the methods disclosed herein can be either fetal or maternal in origin (that is, can have the genotype of either the fetus or mother, respectively). Preferably, the isolated placental cells and populations of isolated placental cells are fetal in origin. As used herein, the phrase "fetal in origin" or "non-maternal in origin" indicates that the isolated placental cells or populations of isolated placental cells are obtained from the umbilical cord or placental structures associated with the fetus, *i.e.*, that have the fetal genotype. As used herein, the phrase "maternal in origin" indicates that the cells or populations of cells are obtained from a placental structures associated with the mother, *e*.*g*., which have the maternal genotype. Isolated placental cells, *e*.*g*., PDACs, or populations of cells comprising the isolated placental cells, can comprise isolated placental cells that are solely fetal or maternal in origin, or can comprise a mixed population of isolated placental cells of both fetal and maternal origin. The isolated placental cells, and populations of cells comprising the isolated placental cells, can be identified and selected by the morphological, marker, and culture characteristics discussed below.

In certain embodiments, if it is determined that the cells used in the methods described herein produce a marker that acts as a surrogate for potency of the cells, then the original non-cultured population of cells is divided into a lot of cells suitable for administration to human subjects. In a specific embodiment, the lot is cryopreserved.

In certain embodiments, if it is determined that the cells used in the methods described herein produce a marker that acts as a surrogate for potency of the cells, then cells corresponding to the population of cells (e.g., other cells from the same lot of cells) are administered to a subject, e.g., a human.

### 5.1.1 Physical and Morphological Characteristics

The isolated placental cells that can be used in the methods described herein (PDACs), when cultured in primary cultures or in cell culture, adhere to the tissue culture substrate, *e*.*g*., tissue culture container surface (*e*.*g*., tissue culture plastic), or to a tissue culture surface coated with extracellular matrix or ligands such as laminin, collagen (*e*.*g*., native or denatured), gelatin, fibronectin, omithine, vitronectin, and extracellular membrane protein (*e*.*g*., MATRIGEL^{®} (BD Discovery Labware, Bedford, Mass.)). The isolated placental cells in culture assume a generally fibroblastoid, stellate appearance, with a number of cytoplasmic processes extending from the central cell body. The cells are, however, morphologically distinguishable from fibroblasts cultured under the same conditions, as the isolated placental cells exhibit a greater number of such processes than do fibroblasts. Morphologically, isolated placental cells are also distinguishable from hematopoietic stem cells, which generally assume a more rounded, or cobblestone, morphology in culture.

In certain embodiments, the isolated placental cells useful in the methods disclosed herein, when cultured in a growth medium, develop embryoid-like bodies. Embryoid-like bodies are noncontiguous clumps of cells that can grow on top of an adherent layer of proliferating isolated placental cells. The term "embryoid-like" is used because the clumps of cells resemble embryoid bodies, clumps of cells that grow from cultures of embryonic stem cells. Growth medium in which embryoid-like bodies can develop in a proliferating culture of isolated placental cells includes medium comprising, *e.g.*, DMEM-LG (*e.g.*, from Gibco); 2% fetal calf serum (*e*.*g*., from Hyclone Labs.); 1x insulin-transferrin-selenium (ITS); 1x linoleic acid-bovine serum albumin (LA-BSA); 10⁻⁹ M dexamethasone (*e*.*g*., from Sigma); 10⁻⁴ M ascorbic acid 2-phosphate (*e.g.*, from Sigma); epidermal growth factor 10 ng/mL (*e.g.*, from R&D Systems); and platelet-derived growth factor (PDGF-BB) 10 ng/mL (*e*.*g*., from R&D Systems).

### 5.1.2 Cell Surface, Molecular and Genetic Markers

The isolated placental cells, *e*.*g*., isolated multipotent placental cells or isolated placental stem cells, and populations of such isolated placental cells, useful in the methods disclosed herein, are tissue culture plastic-adherent human placental cells that have characteristics of multipotent cells or stem cells, and express a plurality of markers that can be used to identify and/or isolate the cells, or populations of cells that comprise the stem cells. In certain embodiments, the PDACs are angiogenic, *e.g.*, *in vitro* or *in vivo.* The isolated placental cells, and placental cell populations described herein (that is, two or more isolated placental cells) include placental cells and placental cell-containing cell populations obtained directly from the placenta, or any part thereof (*e*.*g*., chorion, placental cotyledons, or the like). Isolated placental cell populations also include populations of (that is, two or more) isolated placental cells in culture, and a population in a container, *e*.*g*., a bag. The isolated placental cells useful in the methods described herein are not bone marrow-derived mesenchymal cells, adipose-derived mesenchymal stem cells, or mesenchymal cells obtained from umbilical cord blood, placental blood, or peripheral blood. Placental cells, *e*.*g*., placental multipotent cells and placental cells, useful in the methods described herein are described herein and, *e*.*g*., in U.S. Patent Nos. 7,311,904; 7,31 1,905; and 7,468,276; and in U.S. Patent Application Publication No. 2007/0275362.

In one embodiment, the isolated placental cells, e.g, PDACs, are CD34⁻, CD10⁺ and CD105⁺ as detected by flow cytometry. In another specific embodiment, the isolated CD34⁻, CD10⁺, CD105⁺ placental cells have the potential to differentiate into cells of a neural phenotype, cells of an osteogenic phenotype, and/or cells of a chondrogenic phenotype. In another specific embodiment, the isolated CD34⁻, CD10⁺, CD105⁺ placental cells are additionally CD200⁺. In another specific embodiment, the isolated CD34⁻, CD10⁺, CD105⁺ placental cells are additionally CD45⁻ or CD90⁺. In another specific embodiment, the isolated CD34⁻, CD10⁺, CD105⁺ placental cells are additionally CD45⁻ and CD90⁺, as detected by flow cytometry. In another specific embodiment, the isolated CD34⁻, CD10⁺, CD105⁺, CD200⁺ placental cells are additionally CD90⁺ or CD45⁻, as detected by flow cytometry. In another specific embodiment, the i solated CD34⁻, CD10⁺, CD105⁺, CD200⁺ placental cells are additionally CD90⁺ and CD45⁻, as detected by flow cytometry, *i.e.*, the cells are CD34⁻, CD10⁺, CD45⁻, CD90⁺, CD105⁺ and CD200⁺. In another specific embodiment, said CD34⁻, CD10⁺, CD45⁻, CD90⁺, CD105⁺, CD200⁺ cells are additionally CD80⁻ and CD86⁻.

In certain embodiments, said placental cells are CD34⁻, CD10⁺, CD105⁺ and CD200⁺, and one or more of CD38⁻, CD45⁻, CD80⁻, CD86⁻, CD133⁻, HLA-DR,DP,DQ⁻, SSEA3⁻, SSEA4⁻, CD29⁺, CD44⁺, CD73⁺, CD90⁺, CD105⁺, HLA-A,B,C⁺, PDL1⁺, ABC-p⁺, and/or OCT-4⁺, as detected by flow cytometry. In other embodiments, any of the CD34⁻, CD10⁺, CD105⁺ cells described above are additionally one or more of CD29⁺, CD38⁻, CD44⁺, CD54⁺, SH3⁺ or SH4⁺. In another specific embodiment, the cells are additionally CD44⁺. In another specific embodiment of any of the isolated CD34⁻, CD10⁺, CD105⁺ placental cells above, the cells are additionally one or more of CD117⁻, CD133⁻, KDR⁻ (VEGFR2⁻), HLA-A,B,C⁺, HLA-DP,DQ,DR⁻, or Programmed Death-1 Ligand (PDL1)⁺, or any combination thereof.

In another embodiment, the CD34-, CD10+, CD105+ cells are additionally one or more of CD13+, CD29+, CD33+, CD38-, CD44+, CD45-, CD54+, CD62E-, CD62L-, CD62P-, SH3+ (CD73+), SH4+ (CD73+), CDSO-, CD86-, CD90+, SH2+ (CD105+), CD106/VCAM+, CD117-, CD144/VE-cadherinlow, CD184/CXCR4-, CD200+, CD133-, OCT-4+, SSEA3-, SSEA4-, ABC-p+, KDR- (VEGFR2-), HLA-A,B,C+, HLA-DP,DQ,DR-, HLA-G-, or Programmed Death-1 Ligand (PDL1)+, or any combination thereof. In a other embodiment, the CD34-, CD10+, CD105+ cells are additionally CD13+, CD29+, CD33+, CD38-, CD44+, CD45-, CD54/ICAM+, CD62E-, CD62L-, CD62P-, SH3+ (CD73+), SH4+ (CD73+), CDSO-, CD86-, CD90+, SH2+ (CD105+), CD106/VCAM+, CD117-, CD144/VE-cadherinlow, CD184/CXCR4-, CD200+, CD133-, OCT-4+, SSEA3-, SSEA4-, ABC-p+, KDR-(VEGFR2-), HLA-A,B,C+, HLA-DP,DQ,DR-, HLA-G-, and Programmed Death-1 Ligand (PDL1)+.

In another specific embodiment, any of the placental cells described herein are additionally ABC-p+, as detected by flow cytometry, or OCT-4+ (POU5F1+), as determined by RT-PCR, wherein ABC-p is a placenta-specific ABC transporter protein (also known as breast cancer resistance protein (BCRP) and as mitoxantrone resistance protein (MXR)), and OCT-4 is the Octamer-4 protein (POUSF1). In another specific embodiment, any of the placental cells described herein are additionally SSEA3- or SSEA4-, as determined by flow cytometry, wherein SSEA3 is Stage Specific Embryonic Antigen 3, and SSEA4 is Stage Specific Embryonic Antigen 4. In another specific embodiment, any of the placental cells described herein are additionally SSEA3- and SSEA4-.

In another specific embodiment, any of the placental cells described herein are additionally one or more of MHC-I+ (e.g., HLA-A,B,C+), MHC-II- (e.g., HLA-DP,DQ,DR-) or HLA-G-. In another specific embodiment, any of the placental cells described herein are additionally one or more of MHC-I+ (e.g., HLA-A,B,C+), MHC-II- (e.g., HLA-DP,DQ,DR-) and HLA-G-.

In certain embodiments, the isolated placental cells useful in the methods described herein are one or more, or all, of CD10+, CD29+, CD34-, CD38-, CD44+, CD45-, CD54+, CD90+, SH2+, SH3+, SH4+, SSEA3-, SSEA4-, OCT-4+, and ABC-p+, wherein said isolated placental cells are obtained by physical and/or enzymatic disruption of placental tissue. In a specific embodiment, the isolated placental cells are OCT-4+ and ABC-p+. In another specific embodiment, the isolated placental cells are OCT-4+ and CD34-, wherein said isolated placental cells have at least one of the following characteristics: CD10+, CD29+, CD44+, CD45-, CD54+, CD90+, SH3+, SH4+, SSEA3-, and SSEA4-. In another specific embodiment, the isolated placental cells are OCT-4+, CD34-, CD10+, CD29+, CD44+, CD45-, CD54+, CD90+, SH3+, SH4+, SSEA3-, and SSEA4-. In another embodiment, the isolated placental cells are OCT-4+, CD34-, SSEA3-, and SSEA4-. In another specific embodiment, the isolated placental cells are OCT-4+ and CD34-, and is either SH2+ or SH3+. In another specific embodiment, the isolated placental cells are OCT-4+, CD34-, SH2+, and SH3+. In another specific embodiment, the isolated placental cells are OCT-4+, CD34-, SSEA3-, and SSEA4-, and are either SH2+ or SH3+. In another specific embodiment, the isolated placental cells are OCT-4+ and CD34-, and either SH2+ or SH3+, and is at least one of CD10+, CD29+, CD44+, CD45-, CD54+, CD90+, SSEA3-, or SSEA4-. In another specific embodiment, the isolated placental cells are OCT-4+, CD34-, CD10+, CD29+, CD44+, CD45-, CD54+, CD90+, SSEA3-, and SSEA4-, and either SH2+ or SH3+.

In another embodiment, the isolated placental cells useful in the methods disclosed herein are SH2+, SH3+, SH4+ and OCT-4+. In another specific embodiment, the isolated placental cells are CD10+, CD29+, CD44+, CD54+, CD90+, CD34-, CD45-, SSEA3-, or SSEA4-. In another embodiment, the isolated placental cells are SH2+, SH3+, SH4+, SSEA3- and SSEA4-. In another specific embodiment, the isolated placental cells are SH2+, SH3+, SH4+, SSEA3- and SSEA4-, CD10+, CD29+, CD44+, CD54+, CD90+, OCT-4+, CD34- or CD45-.

In another embodiment, the isolated placental cells useful in the methods disclosed herein are CD10+, CD29+, CD34-, CD44+, CD45-, CD54+, CD90+, SH2+, SH3+, and SH4+; wherein said isolated placental cells are additionally one or more of OCT-4+, SSEA3- or SSEA4-.

In certain embodiments, isolated placental cells useful in the methods disclosed herein are CD200+ or HLA-G-. In a specific embodiment, the isolated placental cells are CD200+ and HLA-G-. In another specific embodiment, the isolated placental cells are additionally CD73+ and CD105+. In another specific embodiment, the isolated placental cells are additionally CD34-, CD38- or CD45-. In another specific embodiment, the isolated placental cells are additionally CD34-, CD38- and CD45-. In another specific embodiment, said stem cells are CD34-, CD38-, CD45-, CD73+ and CD105+. In another specific embodiment, said isolated CD200+ or HLA-G- placental cells facilitate the formation of embryoid-like bodies in a population of placental cells comprising the isolated placental cells, under conditions that allow the formation of embryoid-like bodies. In another specific embodiment, the isolated placental cells are isolated away from placental cells that are not stem or multipotent cells. In another specific embodiment, said isolated placental cells are isolated away from placental cells that do not display these markers.

In another embodiment, the isolated placental cells useful in the methods described herein are CD73+, CD105+, and CD200+. In another specific embodiment, the isolated placental cells are HLA-G-. In another specific embodiment, the isolated placental cells are CD34-, CD38- or CD45-. In another specific embodiment, the isolated placental cells are CD34-, CD38- and CD45-. In another specific embodiment, the isolated placental cells are CD34-, CD38-, CD45-, and HLA-G-. In another specific embodiment, the isolated CD73+, CD105+, and CD200+ placental cells facilitate the formation of one or more embryoid-like bodies in a population of placental cells comprising the isolated placental cells, when the population is cultured under conditions that allow the formation of embryoid-like bodies. In another specific embodiment, the isolated placental cells are isolated away from placental cells that are not the isolated placental cells. In another specific embodiment, the isolated placental cells are isolated away from placental cells that do not display these markers.

In certain other embodiments, the isolated placental cells are one or more of CD10+, CD29+, CD34-, CD38-, CD44+, CD45-, CD54+, CD90+, SH2+, SH3+, SH4+, SSEA3-, SSEA4-, OCT-4+, HLA-G- or ABC-p+. In a specific embodiment, the isolated placental cells are CD10+, CD29+, CD34-, CD38-, CD44+, CD45-, CD54+, CD90+, SH2+, SH3+, SH4+, SSEA3-, SSEA4-, and OCT-4+. In another specific embodiment, the isolated placental cells are CD10+, CD29+, CD34-, CD38-, CD45-, CD54+, SH2+, SH3+, and SH4+. In another specific embodiment, the isolated placental cells are CD10+, CD29+, CD34-, CD38-, CD45-, CD54+, SH2+, SH3+, SH4+ and OCT-4+. In another specific embodiment, the isolated placental cells are CD10+, CD29+, CD34-, CD38-, CD44+, CD45-, CD54+, CD90+, HLA-G-, SH2+, SH3+, SH4+. In another specific embodiment, the isolated placental cells are OCT-4+ and ABC-p+. In another specific embodiment, the isolated placental cells are SH2+, SH3+, SH4+ and OCT-4+. In another embodiment, the isolated placental cells are OCT-4+, CD34-, SSEA3-, and SSEA4-. In a specific embodiment, said isolated OCT-4+, CD34-, SSEA3-, and SSEA4- placental cells are additionally CD10+, CD29+, CD34-, CD44+, CD45-, CD54+, CD90+, SH2+, SH3+, and SH4+. In another embodiment, the isolated placental cells are OCT-4+ and CD34-, and either SH3+ or SH4+. In another embodiment, the isolated placental cells are CD34- and either CD10+, CD29+, CD44+, CD54+, CD90+, or OCT-4+.

In another embodiment, the isolated placental cells useful in the methods described herein are CD200+ and OCT-4+. In a specific embodiment, the isolated placental cells are CD73+ and CD105+. In another specific embodiment, said isolated placental cells are HLA-G-. In another specific embodiment, said isolated CD200+, OCT-4+ placental cells are CD34-, CD38- or CD45-. In another specific embodiment, said isolated CD200+, OCT-4+ placental cells are CD34-, CD38- and CD45-. In another specific embodiment, said isolated CD200+, OCT-4+ placental cells are CD34-, CD38-, CD45-, CD73+, CD105+ and HLA-G-. In another specific embodiment, the isolated CD200+, OCT-4+ placental cells facilitate the production of one or more embryoid-like bodies by a population of placental cells that comprises the isolated cells, when the population is cultured under conditions that allow the formation of embryoid-like bodies. In another specific embodiment, said isolated CD200+, OCT-4+ placental cells are isolated away from placental cells that are not stem cells. In another specific embodiment, said isolated CD200+, OCT-4+ placental cells are isolated away from placental cells that do not display these characteristics.

In another embodiment, the isolated placental cells useful in the methods described herein are CD73+, CD105+ and HLA-G-. In another specific embodiment, the isolated CD73+, CD105+ and HLA-G- placental cells are additionally CD34-, CD38- or CD45-. In another specific embodiment, the isolated CD73+, CD105+, HLA-G- placental cells are additionally CD34-, CD38- and CD45-. In another specific embodiment, the isolated CD73+, CD105+, HLA-G- placental cells are additionally OCT-4+. In another specific embodiment, the isolated CD73+, CD105+, HLA-G- placental cells are additionally CD200+. In another specific embodiment, the isolated CD73+, CD105+, HLA-G- placental cells are additionally CD34-, CD38-, CD45-, OCT-4+ and CD200+. In another specific embodiment, the isolated CD73+, CD105+, HLA-G- placental cells facilitate the formation of embryoid-like bodies in a population of placental cells comprising said cells, when the population is cultured under conditions that allow the formation of embryoid-like bodies. In another specific embodiment, said the isolated CD73+, CD105+, HLA-G- placental cells are isolated away from placental cells that are not the isolated CD73+, CD105+, HLA-G- placental cells. In another specific embodiment, said the isolated CD73+, CD105+, HLA-G- placental cells are isolated away from placental cells that do not display these markers.

In another embodiment, the isolated placental cells useful in the methods described herein are CD73+ and CD105+ and facilitate the formation of one or more embryoid-like bodies in a population of isolated placental cells comprising said CD73+, CD105+ cells when said population is cultured under conditions that allow formation of embryoid-like bodies. In another specific embodiment, said isolated CD73+, CD105+ placental cells are additionally CD34-, CD38- or CD45-. In another specific embodiment, said isolated CD73+, CD105+ placental cells are additionally CD34-, CD38- and CD45-. In another specific embodiment, said isolated CD73+, CD105+ placental cells are additionally OCT-4+. In another specific embodiment, said isolated CD73+, CD105+ placental cells are additionally OCT-4+, CD34-, CD38- and CD45-. In another specific embodiment, said isolated CD73+, CD105+ placental cells are isolated away from placental cells that are not said cells. In another specific embodiment, said isolated CD73+, CD105+ placental cells are isolated away from placental cells that do not display these characteristics.

In another embodiment, the isolated placental cells useful in the methods described herein are OCT-4+ and facilitate formation of one or more embryoid-like bodies in a population of isolated placental cells comprising said cells when cultured under conditions that allow formation of embryoid-like bodies. In a specific embodiment, said isolated OCT-4+ placental cells are additionally CD73+ and CD105+. In another specific embodiment, said isolated OCT-4+ placental cells are additionally CD34-, CD38-, or CD45-. In another specific embodiment, said isolated OCT-4+ placental cells are additionally CD200+. In another specific embodiment, said isolated OCT-4+ placental cells are additionally CD73+, CD105+, CD200+, CD34-, CD38-, and CD45-. In another specific embodiment, said isolated OCT-4+ placental cells are isolated away from placental cells that are not OCT-44- placental cells. In another specific embodiment, said isolated OCT-4+ placental cells are isolated away from placental cells that do not display these characteristics.

In another embodiment, the isolated placental cells useful in the methods described herein are isolated HLA-A,B,C+, CD45-, CD133- and CD34- placental cells. In another embodiment, a cell population useful in the methods described herein is a population of cells comprising isolated placental cells, wherein at least about 70%, at least about 80%, at least about 90%, at least about 95% or at least about 99% of cells in said isolated population of cells are isolated HLA-A,B,C4-, CD45-, CD133- and CD34- placental cells. In a specific embodiment, said isolated placental cell or population of isolated placental cells is isolated away from placental cells that are not HLA-A,B,C4-, CD45-, CD133- and CD34- placental cells. In another specific embodiment, said isolated placental cells are non-maternal in origin. In another specific embodiment, said isolated population of placental cells are substantially free of maternal components; e.g., at least about 40%, 45%, 5-0%, 55%, 60%, 65%, 70%, 75%, 90%, 85%, 90%, 95%, 98% or 99% of said cells in said isolated population of placental cells are non-maternal in origin.

In another embodiment, the isolated placental cells useful in the methods described herein are isolated CD10+, CD13+, CD33+, CD45-, CD117- and CD133- placental cells. In another embodiment, a cell population useful in the methods described herein is a population of cells comprising isolated placental cells, wherein at least about 70%, at least about 80%, at least about 90%, at least about 95% or at least about 99% of cells in said population of cells are isolated CD10+, CD13+, CD33+, CD45-, CD117- and CD133- placental cells. In a specific embodiment, said isolated placental cells or population of isolated placental cells is isolated away from placental cells that are not said isolated placental cells. In another specific embodiment, said isolated CD10+, CD13+, CD33+, CD45-, CD117- and CD133- placental cells are non-maternal in origin, i.e., have the fetal genotype. In another specific embodiment, at least about 40%, 45%, 50%, 55%, 60%, 65%, 70%, 75%, 90%, 85%, 90%, 95%, 98% or 99% of said cells in said isolated population of placental cells, are non-maternal in origin. In another specific embodiment, said isolated placental cells or population of isolated placental cells are isolated away from placental cells that do not display these characteristics.

In another embodiment, the isolated placental cells useful in the methods described herein are isolated CD10-, CD33-, CD44+, CD45-, and CD117- placental cells. In another embodiment, a cell population useful for the in the methods described herein is a population of cells comprising, e.g., enriched for, isolated placental cells, wherein at least about 70%, at least about 80%, at least about 90%, at least about 95% or at least about 99% of cells in said population of cells are isolated CD10-, CD33-, CD44+, CD45-, and CD117- placental cells. In a specific embodiment, said isolated placental cell or population of isolated placental cells is isolated away from placental cells that are not said cells. In another specific embodiment, said isolated placental cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental cell or population of isolated placental cells is isolated away from placental cells that do not display these markers.

In another embodiment, the isolated placental cells useful in the methods described herein are isolated CD10-, CD13-, CD33-, CD45-, and CD117- placental cells. In another embodiment, a cell population useful for in the methods described herein is a population of cells comprising, e.g., enriched for, isolated CD10-, CD13-, CD33-, CD45-, and CD117- placental cells, wherein at least about 70%, at least about 80%, at least about 90%, at least about 95% or at least about 99% of cells in said population are CD10-, CD13-, CD33-, CD45-, and CD117-placental cells. In a specific embodiment, said isolated placental cells or population of isolated placental cells are isolated away from placental cells that are not said cells. In another specific embodiment, said isolated placental cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental cells or population of isolated placental cells is isolated away from placental cells that do not display these characteristics.

In another embodiment, the isolated placental cells useful in the methods described herein are HLA A,B,C+, CD45-, CD34-, and CD133-, and are additionally CD10+, CD13+, CD38+, CD44+, CD90+, CD105+, CD200+ and/or HLA-G-, and/or negative for CD117. In another embodiment, a cell population useful in the methods described herein is a population of cells comprising isolated placental cells, wherein at least about 20%, 25%, 30%, 35%, 40%, 45%, 50%, 55%, 60%, 65%, 70%, 75%, 80%, 85%, 90%, 95%, 98% or about 99% of the cells in said population are isolated placental cells that are HLA A,B,C-, CD45-, CD34-, CD133-, and that are additionally positive for CD10, CD13, CD38, CD44, CD90, CD105, CD200, and/or negative for CD117 and/or HLA-G. In a specific embodiment, said isolated placental cells or population of isolated placental cells are isolated away from placental cells that are not said cells. In another specific embodiment, said isolated placental cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental cells or population of isolated placental cells are isolated away from placental cells that do not display these markers.

In another embodiment, the isolated placental cells useful in the methods described herein are isolated placental cells that are CD200+ and CD10+, as determined by antibody binding, and CD117-, as determined by both antibody binding and RT-PCR. In another embodiment, the isolated placental cells useful in the methods described herein are isolated placental cells, e.g., placental stem cells or placental multipotent cells, that are CD10+, CD29-, CD54+, CD200+, HLA-G-, MHC class I+ and β-2-microglobulin+, In another embodiment, isolated placental cells useful in the methods described herein are placental cells wherein the expression of at least one cellular marker is at least two-fold higher than for a mesenchymal stem cell (e.g., a bone marrow-derived mesenchymal stem cell). In another specific embodiment, said isolated placental cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin.

In another embodiment, the isolated placental cells useful in the methods described herein are isolated placental cells, e.g., placental stem cells or placental multipotent cells, that are one or more of CD10+, CD29+, CD44+, CD45-, CD54/ICAM+, CD62E-, CD62L-, CD62P-, CDSO-, CD86-, CD103-, CD104-, CD105+, CD106/VCAM+, CD144/VE-cadherinlow, CD184/CXCR4-, β2-microglobulinlow, MHC-Ilow, MHC-II-, HLA-Glow, and/or PDL1low. In a specific embodiment, the isolated placental cells are at least CD29+ and CD54+. In another specific embodiment, the isolated placental cells are at least CD44+ and CD106+. In another specific embodiment, the isolated placental cells are at least CD29+.

In another embodiment, a cell population useful in the methods described herein comprises isolated placental cells, and at least 50%, 60%, 70%, 80%, 90%, 95%, 98% or 99% of the cells in said cell population are isolated placental cells that are one or more of CD10+, CD29+, CD44+, CD45-, CD54/ICAM+, CD62-E-, CD62-L-, CD62-P-, CDSO-, CD86-, CD103-, CD104-, CD105+, CD106/VCAM+, CD144/VE-cadherindim, CD184/CXCR4-, β2-microglobulindim, HLA-Idim, HLA-II-, HLA-Gdim, and/or PDL1dim. In another specific embodiment, at least 50%, 60%, 70%, 80%, 90%, 95%, 98% or 99% of cells in said cell population are CD10+, CD29+, CD44+, CD45-, CD54/ICAM+, CD62-E-, CD62-L-, CD62-P-, CD80-, CD86-, CD103-, CD104-, CD105+, CD106/VCAM+, CD144/VE-cadherindim, CD1S4/CXCR4-, β2-microglobulindim, MHC-Idim, MHC-II-, HLA-Gdim, and PDL1dim.

In another embodiment, the isolated placental cells useful in the methods described herein are isolated placental cells that are one or more, or all, of CD10+, CD29+, CD34-, CD38-, CD44+, CD45-, CD54+, CD90+, SH2+, SH3+, SH4+, SSEA3-, SSEA4-, OCT-4+, and ABC-p+, where ABC-p is a placenta-specific ABC transporter protein (also known as breast cancer resistance protein (BCRP) and as mitoxantrone resistance protein (MXR)), wherein said isolated placental cells are obtained by perfusion of a mammalian, e.g., human, placenta that has been drained of cord blood and perfused to remove residual blood.

In another specific embodiment of any of the above characteristics, expression of the cellular marker (e.g., cluster of differentiation or immunogenic marker) is determined by flow cytometry; in another specific embodiment, expression of the marker is determined by RT-PCR.

Gene profiling confirms that isolated placental cells, and populations of isolated placental cells, are distinguishable from other cells, e.g., mesenchymal stem cells, e.g., bone marrow-derived mesenchymal stem cells. The isolated placental cells useful in the methods described herein can be distinguished from, e.g., mesenchymal stem cells on the basis of the expression of one or more genes, the expression of which is significantly higher in the isolated placental cells, or in certain isolated umbilical cord stem cells, in comparison to bone marrow-derived mesenchymal stem cells. In particular, the isolated placental cells, useful in the methods provided herein, can be distinguished from mesenchymal stem cells on the basis of the expression of one or more genes, the expression of which is significantly higher (that is, at least twofold higher) in the isolated placental cells than in an equivalent number of bone marrow-derived mesenchymal stem cells, wherein the one or more genes are ACTG2, ADARB1, AMIGO2, ARTS-1, B4GALT6, BCHE, C11orf9, CD200, COL4A1, COL4A2, CPA4, DMD, DSC3, DSG2, ELOVL2, F2RL1, FLJ10781, GATA6, GPR126, GPRC5B, HLA-G, ICAM1, IER3, IGFBP7, IL1A, IL6, IL18, KRT18, KRT8, LIPG, LRAP, MATN2, MEST, NFE2L3, NUAK1, PCDH7, PDLIM3, PKP2, RTN1, SERPINB9, ST3GAL6, ST6GALNAC5, SLC12A8, TCF21, TGFB2, VTN, ZC3H12A, or a combination of any of the foregoing, when the cells are grown under equivalent conditions. See, e.g., U.S. Patent Application Publication No. 2007/0275362.

In certain specific embodiments, said expression of said one ore more genes is determined, e.g., by RT-PCR or microarray analysis, e.g, using a U133-A microarray (Affymetrix). In another specific embodiment, said isolated placental cells express said one or more genes when cultured for a number of population doublings, e.g., anywhere from about 3 to about 35 population doublings, in a medium comprising DMEM-LG (e.g., from Gibco); 2% fetal calf serum (e.g., from Hyclone Labs.); 1x insulin-transferrin-selenium (ITS); 1x linoleic acid-bovine serum albumin (LA-BSA); 10-9 M dexamethasone (e.g., from Sigma); 10-4 M ascorbic acid 2-phosphate (e.g., from Sigma); epidermal growth factor 10 ng/mL (e.g., from R&D Systems); and platelet-derived growth factor (PDGF-BB) 10 ng/mL (e.g., from R&D Systems). In another specific embodiment, the isolated placental cell-specific or isolated umbilical cord cell-specific gene is CD200.

Specific sequences for these genes can be found in GenBank at accession nos. NM_001615 (ACTG2), BC065545 (ADARB1), (NM_181847 (AMIGO2), AY358590 (ARTS-1), BC074884 (B4GALT6), BC008396 (BCHE), BC020196 (C11orf9), BC031103 (CD200), NM_001845 (COL4A1), NM_001846 (COL4A2), BC052289 (CPA4), BC094758 (DMD), AF293359 (DSC3), NM_001943 (DSG2), AF338241 (ELOVL2), AY336105 (F2RL1), NM_018215 (FLJ10781), AY416799 (GATA6), BC075798 (GPR126), NM_016235 (GPRC5B), AF340038 (ICAM1), BC000844 (IER3), BC066339 (IGFBP7), BC013142 (IL1A), BT019749 (IL6), BC007461 (IL18), (BC072017) KRT18, BC075839 (KRT8), BC060825 (LIPG), BC065240 (LRAP), BC010444 (MATN2), BC011908 (MEST), BC068455 (NFE2L3), NM_014840 (NUAK1), AB006755 (PCDH7), NM_014476 (PDLIM3), BC126199 (PKP-2), BC090862 (RTN1), BC002538 (SERPINB9), BC023312 (ST3GAL6), BC001201 (ST6GALNAC5), BC126160 or BC065328 (SLC12A8), BC025697 (TCF21), BC096235 (TGFB2), BC005046 (VTN), and BC005001 (ZC3H12A) as of March 2008.

In certain specific embodiments, said isolated placental cells express each of ACTG2, ADARB1, AMIGO2, ARTS-1, B4GALT6, BCHE, C11orf9, CD200, COL4A1, COL4A2, CPA4, DMD, DSC3, DSG2, ELOVL2, F2RL1, FLJ10781, GATA6, GPR126, GPRC5B, HLA-G, ICAM1, IER3, IGFBP7, IL1A, IL6, IL18, KRT18, KRT8, LIPG, LRAP, MATN2, MEST, NFE2L3, NUAK1, PCDH7, PDLIM3, PKP2, RTN1, SERPINB9, ST3GAL6, ST6GALNAC5, SLC12A8, TCF21, TGFB2, VTN, and ZC3H12A at a detectably higher level than an equivalent number of bone marrow-derived mesenchymal stem cells, when the cells are grown under equivalent conditions.

In specific embodiments, the placental cells express CD200 and ARTS1 (aminopeptidase regulator of type 1 tumor necrosis factor); ARTS-1 and LRAP (leukocyte-derived arginine aminopeptidase); IL6 (interleukin-6) and TGFB2 (transforming growth factor, beta 2); IL6 and KRT18 (keratin 18); IER3 (immediate early response 3), MEST (mesoderm specific transcript homolog) and TGFB2; CD200 and IER3; CD200 and IL6; CD200 and KRT18; CD200 and LRAP; CD200 and MEST; CD200 and NFE2L3 (nuclear factor (erythroid-derived 2)-like 3); or CD200 and TGFB2 at a detectably higher level than an equivalent number of bone marrow-derived mesenchymal stem cells (BM-MSCs) wherein said bone marrow-derived mesenchymal stem cells have undergone a number of passages in culture equivalent to the number of passages said isolated placental cells have undergone. In other specific embodiments, the placental cells express ARTS-1, CD200, IL6 and LRAP; ARTS-1, IL6, TGFB2, IER3, KRT18 and MEST; CD200, IER3, IL6, KRT18, LRAP, MEST, NFE2L3, and TGFB2; ARTS-1, CD200, IER3, IL6, KRT18, LRAP, MEST, NFE2L3, and TGFB2; or IER3, MEST and TGFB2 at a detectably higher level than an equivalent number of bone marrow-derived mesenchymal stem cells BM-MSCs, wherein said bone marrow-derived mesenchymal stem cells have undergone a number of passages in culture equivalent to the number of passages said isolated placental cells have undergone.

Expression of the above-referenced genes can be assessed by standard techniques. For example, probes based on the sequence of the gene(s) can be individually selected and constructed by conventional techniques. Expression of the genes can be assessed, e.g., on a microarray comprising probes to one or more of the genes, e.g., an Affymetrix GENECHIP^{®} Human Genome U133A 2.0 array, or an Affymetrix GENECHIP^{®} Human Genome U133 Plus 2.0 (Santa Clara, California). Expression of these genes can be assessed even if the sequence for a particular GenBank accession number is amended because probes specific for the amended sequence can readily be generated using well-known standard techniques.

The level of expression of these genes can be used to confirm the identity of a population of isolated placental cells, to identify a population of cells as comprising at least a plurality of isolated placental cells, or the like. Populations of isolated placental cells, the identity of which is confirmed, can be clonal, e.g., populations of isolated placental cells expanded from a single isolated placental cell, or a mixed population of stem cells, e.g., a population of cells comprising solely isolated placental cells that are expanded from multiple isolated placental cells, or a population of cells comprising isolated placental cells, as described herein, and at least one other type of cell.

The level of expression of these genes can be used to select populations of isolated placental cells. For example, a population of cells, e.g., clonally-expanded cells, may be selected if the expression of one or more of the genes listed above is significantly higher in a sample from the population of cells than in an equivalent population of mesenchymal stem cells. Such selecting can be of a population from a plurality of isolated placental cell populations, from a plurality of cell populations, the identity of which is not known, etc.

Isolated placental cells can be selected on the basis of the level of expression of one or more such genes as compared to the level of expression in said one or more genes in, e.g., a mesenchymal stem cell control, for example, the level of expression in said one or more genes in an equivalent number of bone marrow-derived mesenchymal stem cells. In one embodiment, the level of expression of said one or more genes in a sample comprising an equivalent number of mesenchymal stem cells is used as a control. In another embodiment, the control, for isolated placental cells tested under certain conditions, is a numeric value representing the level of expression of said one or more genes in mesenchymal stem cells under said conditions.

In certain embodiments, the placental cells (e.g., PDACs) useful in the methods provided herein, do not express CD34, as detected by immunolocalization, after exposure to 1 to 100 ng/mL VEGF for 4 to 21 days. In a specific embodiment, said placental adherent cells are adherent to tissue culture plastic. In another specific embodiment, said population of cells induce endothelial cells to form sprouts or tube-like structures when cultured in the presence of an angiogenic factor such as vascular endothelial growth factor (VEGF), epithelial growth factor (EGF), platelet derived growth factor (PDGF) or basic fibroblast growth factor (bFGF), e.g., on a substrate such as MATRIGEL^{™}.

In another aspect, the PDACs provided herein, a population of cells, e.g., a population of PDACs, or a population of cells wherein at least about 50%, 60%, 70%, 80%, 90%, 95% or 98% of cells in said isolated population of cells are PDACs, secrete one or more, or all, of VEGF, HGF, IL-8, MCP-3, FGF2, follistatin, G-CSF, EGF, ENA-78, GRO, IL-6, MCP-1, PDGF-BB, TIMP-2, uPAR, or galectin-1, e.g., into culture medium in which the cell, or cells, are grown. In another embodiment, the PDACs express increased levels of CD202b, IL-8 and/or VEGF under hypoxic conditions (e.g., less than about 5% 02) compared to normoxic conditions (e.g., about 20% or about 21% O2).

In another embodiment, any of the PDACS or populations of cells comprising PDACs described herein can cause the formation of sprouts or tube-like structures in a population of endothelial cells in contact with said placental derived adherent cells. In a specific embodiment, the PDACs are co-cultured with human endothelial cells, which form sprouts or tube-like structures, or support the formation of endothelial cell sprouts, e.g., when cultured in the presence of extracellular matrix proteins such as collagen type I and IV, and/or angiogenic factors such as vascular endothelial growth factor (VEGF), epithelial growth factor (EGF), platelet derived growth factor (PDGF) or basic fibroblast growth factor (bFGF), e.g., in or on a substrate such as placental collagen or MATRIGEL^{™} for at least 4 days. In another embodiment, any of the populations of cells comprising placental derived adherent cells, described herein, secrete angiogenic factors such as vascular endothelial growth factor (VEGF), hepatocyte growth factor (HGF), platelet derived growth factor (PDGF), basic fibroblast growth factor (bFGF), or Interleukin-8 (IL-8) and thereby can induce human endothelial cells to form sprouts or tube-like structures when cultured in the presence of extracellular matrix proteins such as collagen type I and IV e.g., in or on a substrate such as placental collagen or MATRIGEL^{™}.

In another embodiment, any of the above populations of cells comprising placental derived adherent cells (PDACs) secretes angiogenic factors. In specific embodiments, the population of cells secretes vascular endothelial growth factor (VEGF), hepatocyte growth factor (HGF), platelet derived growth factor (PDGF), basic fibroblast growth factor (bFGF), and/or interleukin-8 (IL-8). In other specific embodiments, the population of cells comprising PDACs secretes one or more angiogenic factors and thereby induces human endothelial cells to migrate in an in vitro wound healing assay. In other specific embodiments, the population of cells comprising placental derived adherent cells induces maturation, differentiation or proliferation of human endothelial cells, endothelial progenitors, myocytes or myoblasts.

The isolated placental cells described herein display the above characteristics (e.g., combinations of cell surface markers and/or gene expression profiles) in primary culture, or during proliferation in medium comprising, e.g., DMEM-LG (Gibco), 2% fetal calf serum (FCS) (Hyclone Laboratories), 1x insulin-transferrin-selenium (ITS), 1x lenolenic-acid-bovine-serum-albumin (LA-BSA), 10-9 M dexamethasone (Sigma), 10-4M ascorbic acid 2-phosphate (Sigma), epidermal growth factor (EGF)10ng/ml (R&D Systems), platelet derived-growth factor (PDGF-BB) 10ng/ml (R&D Systems), and 100U penicillin/1000U streptomycin.

In certain embodiments of any of the placental cells disclosed herein, the cells are human. In certain embodiments of any of the placental cells disclosed herein, the cellular marker characteristics or gene expression characteristics are human markers or human genes.

In another specific embodiment of said isolated placental cells or populations of cells comprising the isolated placental cells, said cells or population have been expanded, for example, passaged at least, about, or no more than, 1, 2, 3, 4, 5, 6, 7, 8, 9, 10, 11, 12, 13, 14, 15, 16, 17, 18, 19, or 20 times, or more, or proliferated for at least, about, or no more than, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 population doublings. In another specific embodiment of said isolated placental cells or populations of cells comprising the isolated placental cells, said cells or population are primary isolates. In another specific embodiment of the isolated placental cells, or populations of cells comprising isolated placental cells, that are disclosed herein, said isolated placental cells are fetal in origin (that is, have the fetal genotype).

In certain embodiments, said isolated placental cells do not differentiate during culturing in growth medium, i.e., medium formulated to promote proliferation, e.g., during proliferation in growth medium. In another specific embodiment, said isolated placental cells do not require a feeder layer in order to proliferate. In another specific embodiment, said isolated placental cells do not differentiate in culture in the absence of a feeder layer, solely because of the lack of a feeder cell layer.

In another embodiment, cells useful in the methods described herein are isolated placental cells, wherein a plurality of said isolated placental cells are positive for aldehyde dehydrogenase (ALDH), as assessed by an aldehyde dehydrogenase activity assay. Such assays are known in the art (see, e.g., Bostian and Betts, Biochem. J., 173, 787, (1978)). In a specific embodiment, said ALDH assay uses Aldefluor^{®} (Aldagen, Inc., Ashland, Oregon) as a marker of aldehyde dehydrogenase activity. In a specific embodiment, said plurality is between about 3% and about 25% of cells in said population of cells. In another embodiment, provided herein is a population of isolated umbilical cord cells, e.g., multipotent isolated umbilical cord cells, wherein a plurality of said isolated umbilical cord cells are positive for aldehyde dehydrogenase, as assessed by an aldehyde dehydrogenase activity assay that uses Aldefluor^{®} as an indicator of aldehyde dehydrogenase activity. In a specific embodiment, said plurality is between about 3% and about 25% of cells in said population of cells. In another embodiment, said population of isolated placental cells or isolated umbilical cord cells shows at least three-fold, or at least fivefold, higher ALDH activity than a population of bone marrow-derived mesenchymal stem cells having about the same number of cells and cultured under the same conditions.

In certain embodiments of any of the populations of cells comprising the isolated placental cells described herein, the placental cells in said populations of cells are substantially free of cells having a maternal genotype; e.g., at least 40%, 45%, 50%, 55%, 60%, 65%, 70%, 75%, 80%, 85%, 90%, 95%, 98% or 99% of the placental cells in said population have a fetal genotype. In certain other embodiments of any of the populations of cells comprising the isolated placental cells described herein, the populations of cells comprising said placental cells are substantially free of cells having a maternal genotype; e.g., at least 40%, 45%, 50%, 55%, 60%, 65%, 70%, 75%, 80%, 85%, 90%, 95%, 98% or 99% of the cells in said population have a fetal genotype.

In a specific embodiment of any of the above isolated placental cells or cell populations of isolated placental cells, the karyotype of the cells, or at least about 95% or about 99% of the cells in said population, is normal. In another specific embodiment of any of the above placental cells or cell populations, the cells, or cells in the population of cells, are non-maternal in origin.

Isolated placental cells, or populations of isolated placental cells, bearing any of the above combinations of markers, can be combined in any ratio. Any two or more of the above isolated placental cell populations can be combined to form an isolated placental cell population. For example, an population of isolated placental cells can comprise a first population of isolated placental cells defined by one of the marker combinations described above, and a second population of isolated placental cells defined by another of the marker combinations described above, wherein said first and second populations are combined in a ratio of about 1:99, 2:98, 3:97, 4:96, 5:95, 10:90, 20:80, 30:70, 40:60, 50:50, 60:40, 70:30, 80:20, 90:10, 95:5, 96:4, 97:3, 98:2, or about 99:1. In like fashion, any three, four, five or more of the above-described isolated placental cells or isolated placental cells populations can be combined.

Isolated placental cells useful in the methods described herein can be obtained, e.g., by disruption of placental tissue, with or without enzymatic digestion (see Section 5.2.3) or perfusion (see Section 5.2.4). For example, populations of isolated placental cells can be produced according to a method comprising perfusing a mammalian placenta that has been drained of cord blood and perfused to remove residual blood; perfusing said placenta with a perfusion solution; and collecting said perfusion solution, wherein said perfusion solution after perfusion comprises a population of placental cells that comprises isolated placental cells; and isolating a plurality of said isolated placental cells from said population of cells. In a specific embodiment, the perfusion solution is passed through both the umbilical vein and umbilical arteries and collected after it exudes from the placenta. In another specific embodiment, the perfusion solution is passed through the umbilical vein and collected from the umbilical arteries, or passed through the umbilical arteries and collected from the umbilical vein.

In various embodiments, the isolated placental cells, contained within a population of cells obtained from perfusion of a placenta, are at least 50%, 60%, 70%, 80%, 90%, 95%, 99% or at least 99.5% of said population of placental cells. In another specific embodiment, the isolated placental cells collected by perfusion comprise fetal and maternal cells. In another specific embodiment, the isolated placental cells collected by perfusion are at least 50%, 60%, 70%, 80%, 90%, 95%, 99% or at least 99.5% fetal cells.

In another specific embodiment, provided herein is a composition comprising a population of the isolated placental cells, as described herein, collected by perfusion, wherein said composition comprises at least a portion of the perfusion solution used to collect the isolated placental cells.

Isolated populations of the isolated placental cells described herein can be produced by digesting placental tissue with a tissue-disrupting enzyme to obtain a population of placental cells comprising the cells, and isolating, or substantially isolating, a plurality of the placental cells from the remainder of said placental cells. The whole, or any part of, the placenta can be digested to obtain the isolated placental cells described herein. In specific embodiments, for example, said placental tissue can be a whole placenta, an amniotic membrane, chorion, a combination of amnion and chorion, or a combination of any of the foregoing. In other specific embodiment, the tissue-disrupting enzyme is trypsin or collagenase. In various embodiments, the isolated placental cells, contained within a population of cells obtained from digesting a placenta, are at least 50%, 60%, 70%, 80%, 90%, 95%, 99% or at least 99.5% of said population of placental cells.

The isolated populations of placental cells described above, and populations of isolated placental cells generally, can comprise about, at least, or no more than 1 × 10³, 3 × 10³, 5 × 10³, 1 × 10⁴, 3 × 10⁴, 5 × 10⁴, 1 × 10⁵, 3 × 10⁵, 5 × 10⁵, 1 × 10⁶, 3 × 10⁶, 5 × 10⁶, 1 × 10⁷, 3 × 10⁷, 5 × 10⁷, 1 × 10⁸, 3 × 10⁸, 5 × 10⁸, 1 × 10⁹, 5 × 10⁹, or 1 × 10¹⁰ isolated placental cells (e.g., as part of a pharmaceutical composition comprising placental stem cells) or between about 1 × 10³ to 3 × 10³, 3 × 10³ to 5 × 10³, 5 × 10³ to 1 × 10⁴, 1 × 10⁴ to 3 × 10⁴, 3 × 10⁴ to 5 × 10⁴, 5 × 10⁴ to 1 × 10⁵, 1 × 10⁵ to 3 × 10³, 3 × 10⁵ to 5 × 10⁵, 5x 10⁵ to 1 × 10⁶, 1 × 10⁶ to 3 × 10⁶, 3 × 10⁶ to 5 × 10⁶, 5 × 10⁶ to 1 × 10⁷, 1 × 10⁷ to 3 × 10⁷, 3 × 10⁷ to 5 × 10⁷, 5 × 10⁷ to 1 × 10⁸, 1 × 10⁸ to 3 × 10⁸, 3 × 10⁸ to 5 × 10⁸, 5 × 10⁸ to 1 × 10⁹, 1 × 10⁹ to 5 × 10⁹, or 5 × 10⁹ to 1 × 10¹⁰ isolated placental cells (e.g., as part of a pharmaceutical composition comprising placental stem cells). Populations of isolated placental cells useful in the methods described herein comprise at least 50%, 55%, 60%, 65%, 70%, 75%, 80%, 85%, 90%, 95%, 98%, or 99% viable isolated placental cells, *e.g.,* as determined by, e.g., trypan blue exclusion.

### 5.2 METHODS OF OBTAINING ISOLATED PLACENTAL CELLS

### 5.2.1 Stem Cell Collection Composition

Generally, the isolated placental cells described in Section 5.1, above, are obtained from a mammalian placenta using a physiologically-acceptable solution, e.g., a cell collection composition. An exemplary cell collection composition is described in detail in related U.S. Patent Application Publication No. 2007/0190042, entitled "Improved Medium for Collecting Placental Stem Cells and Preserving Organs,".

The cell collection composition can comprise any physiologically-acceptable solution suitable for the collection and/or culture of cells, e.g., the isolated placental cells described herein, for example, a saline solution (e.g., phosphate-buffered saline, Kreb's solution, modified Kreb's solution, Eagle's solution, 0.9% NaCl. etc.), a culture medium (e.g., DMEM, H.DMEM, etc.), and the like.

The cell collection composition can comprise one or more components that tend to preserve isolated placental cells, that is, prevent the isolated placental cells from dying, or delay the death of the isolated placental cells, reduce the number of isolated placental cells in a population of cells that die, or the like, from the time of collection to the time of culturing. Such components can be, e.g., an apoptosis inhibitor (e.g., a caspase inhibitor or JNK inhibitor); a vasodilator (e.g., magnesium sulfate, an antihypertensive drug, atrial natriuretic peptide (ANP), adrenocorticotropin, corticotropin-releasing hormone, sodium nitroprusside, hydralazine, adenosine triphosphate, adenosine, indomethacin or magnesium sulfate, a phosphodiesterase inhibitor, etc.); a necrosis inhibitor (e.g., 2-(1H-Indol-3-yl)-3-pentylamino-maleimide, pyrrolidine dithiocarbamate, or clonazepam); a TNF-α inhibitor; and/or an oxygen-carrying perfluorocarbon (e.g., perfluorooctyl bromide, perfluorodecyl bromide, etc.).

The cell collection composition can comprise one or more tissue-degrading enzymes, e.g., a metalloprotease, a serine protease, a neutral protease, an RNase, or a DNase, or the like. Such enzymes include, but are not limited to, collagenases (e.g., collagenase I, II, III or IV, a collagenase from Clostridium histolyticum, etc.); dispase, thermolysin, elastase, trypsin, LIBERASE, hyaluronidase, and the like.

The cell collection composition can comprise a bacteriocidally or bacteriostatically effective amount of an antibiotic. In certain non-limiting embodiments, the antibiotic is a macrolide (e.g., tobramycin), a cephalosporin (e.g., cephalexin, cephradine, cefuroxime, cefprozil, cefaclor, cefixime or cefadroxil), a clarithromycin, an erythromycin, a penicillin (e.g., penicillin V) or a quinolone (e.g., ofloxacin, ciprofloxacin or norfloxacin), a tetracycline, a streptomycin, etc. In a particular embodiment, the antibiotic is active against Gram(+) and/or Gram(-) bacteria, e.g., Pseudomonas aeruginosa, Staphylococcus aureus, and the like. In one embodiment, the antibiotic is gentamycin, e.g., about 0.005% to about 0.01% (w/v) in culture medium

The cell collection composition can also comprise one or more of the following compounds: adenosine (about 1 mM to about 50 mM); D-glucose (about 20 mM to about 100 mM); magnesium ions (about 1 mM to about 50 mM); a macromolecule of molecular weight greater than 20,000 daltons, in one embodiment, present in an amount sufficient to maintain endothelial integrity and cellular viability (e.g., a synthetic or naturally occurring colloid, a polysaccharide such as dextran or a polyethylene glycol present at about 25 g/l to about 100 g/l, or about 40 g/l to about 60 g/l); an antioxidant (e.g., butylated hydroxyanisole, butylated hydroxytoluene, glutathione, vitamin C or vitamin E present at about 25 µM to about 100 µM); a reducing agent (e.g., N-acetylcysteine present at about 0.1 mM to about 5 mM); an agent that prevents calcium entry into cells (e.g., verapamil present at about 2 µM to about 25 µM); nitroglycerin (e.g., about 0.05 g/L to about 0.2 g/L); an anticoagulant, in one embodiment, present in an amount sufficient to help prevent clotting of residual blood (e.g., heparin or hirudin present at a concentration of about 1000 units/l to about 100,000 units/l); or an amiloride containing compound (e.g., amiloride, ethyl isopropyl amiloride, hexamethylene amiloride, dimethyl amiloride or isobutyl amiloride present at about 1.0 µM to about 5 µM).

### 5.2.2 Collection and Handling of Placenta

Generally, a human placenta is recovered shortly after its expulsion after birth. In a preferred embodiment, the placenta is recovered from a patient after informed consent and after a complete medical history of the patient is taken and is associated with the placenta. Preferably, the medical history continues after delivery. Such a medical history can be used to coordinate subsequent use of the placenta or the isolated placental cells harvested therefrom. For example, isolated human placental cells can be used, in light of the medical history, for personalized medicine for the infant associated with the placenta, or for parents, siblings or other relatives of the infant.

Prior to recovery of isolated placental cells, the umbilical cord blood and placental blood are preferably removed. In certain embodiments, after delivery, the cord blood in the placenta is recovered. The placenta can be subjected to a conventional cord blood recovery process. Typically a needle or cannula is used, with the aid of gravity, to exsanguinate the placenta (see, e.g., Anderson, U.S. Patent No. 5,372,581; Hessel et al., U.S. Patent No. 5,415,665). The needle or cannula is usually placed in the umbilical vein and the placenta can be gently massaged to aid in draining cord blood from the placenta. Such cord blood recovery may be performed commercially, e.g., LifeBank USA, Cedar Knolls, N.J. Preferably, the placenta is gravity drained without further manipulation so as to minimize tissue disruption during cord blood recovery.

Typically, a placenta is transported from the delivery or birthing room to another location, e.g., a laboratory, for recovery of cord blood and collection of stem cells by, e.g., perfusion or tissue dissociation. The placenta is preferably transported in a sterile, thermally insulated transport device (maintaining the temperature of the placenta between 20-28°C), for example, by placing the placenta, with clamped proximal umbilical cord, in a sterile zip-lock plastic bag, which is then placed in an insulated container. In another embodiment, the placenta is transported in a cord blood collection kit substantially as described in pending United States Patent No. 7,147,626.

Preferably, the placenta is delivered to the laboratory four to twenty-four hours following delivery. In certain embodiments, the proximal umbilical cord is clamped, preferably within 4-5 cm (centimeter) of the insertion into the placental disc prior to cord blood recovery. In other embodiments, the proximal umbilical cord is clamped after cord blood recovery but prior to further processing of the placenta.

The placenta, prior to cell collection, can be stored under sterile conditions and at either room temperature or at a temperature of 5°C to 25°C. The placenta may be stored for a period of for a period of four to twenty-four hours, up to forty-eight hours, or longer than forty eight hours, prior to perfusing the placenta to remove any residual cord blood. In one embodiment, the placenta is harvested from between about zero hours to about two hours post-expulsion. The placenta is preferably stored in an anticoagulant solution at a temperature of 5°C to 25°C. Suitable anticoagulant solutions are well known in the art. For example, a solution of heparin or warfarin sodium can be used. In a preferred embodiment, the anticoagulant solution comprises a solution of heparin (e.g., 1% w/w in 1:1000 solution). The exsanguinated placenta is preferably stored for no more than 36 hours before placental cells are collected.

The mammalian placenta or a part thereof, once collected and prepared generally as above, can be treated in any art-known manner, *e.g.*, can be perfused or disrupted, *e.g.*, digested with one or more tissue-disrupting enzymes, to obtain isolated placental cells.

### 5.2.3 Physical Disruption and Enzymatic Digestion of Placental Tissue

Placental cells can be collected from a mammalian placenta by physical disruption of part of all of the organ. For example, the placenta, or a portion thereof, may be, e.g., crushed, sheared, minced, diced, chopped, macerated or the like. The tissue can then be cultured to obtain a population of isolated placental cells. Typically, the placental tissue is disrupted using, e.g., culture medium, a saline solution, or a stem cell collection.

The placenta can be dissected into components prior to physical disruption and/or enzymatic digestion and stem cell recovery. Isolated placental cells can be obtained from all or a portion of the amniotic membrane, chorion, umbilical cord, placental cotyledons, or any combination thereof, including from a whole placenta. Preferably, isolated placental cells are obtained from placental tissue comprising amnion and chorion. Typically, isolated placental cells can be obtained by disruption of a small block of placental tissue, e.g., a block of placental tissue that is about 1, 2, 3, 4, 5, 6, 7, 8, 9, 10, 20, 30, 40, 50, 60, 70, 80, 90, 100, 200, 300, 400, 500, 600, 700, 800, 900 or about 1000 cubic millimeters in volume. Any method of physical disruption can be used, provided that the method of disruption leaves a plurality, more preferably a majority, and more preferably at least 60%, 70%, 80%, 90%, 95%, 98%, or 99% of the cells in said organ viable, as determined by, e.g., trypan blue exclusion.

The isolated adherent placental cells can generally be collected from a placenta, or portion thereof, at any time within about the first three days post-expulsion, but preferably between about 8 hours and about 18 hours post-expulsion.

In a specific embodiment, the disrupted tissue is cultured in tissue culture medium suitable for the proliferation of isolated placental cells.

In another specific embodiment, isolated placental cells are collected by physical disruption of placental tissue, wherein the physical disruption includes enzymatic digestion, which can be accomplished by use of one or more tissue-digesting enzymes. The placenta, or a portion thereof, may also be physically disrupted and digested with one or more enzymes, and the resulting material then immersed in, or mixed into, a cell collection composition.

A preferred cell collection composition comprises one or more tissue-disruptive enzyme(s). Enzymes that can be used to disrupt placenta tissue include papain, deoxyribonucleases, serine proteases, such as trypsin, chymotrypsin, collagenase, dispase or elastase. Serine proteases may be inhibited by alpha 2 microglobulin in serum and therefore the medium used for digestion is usually serum-free. EDTA and DNase are commonly used in enzyme digestion procedures to increase the efficiency of cell recovery. The digestate is preferably diluted so as to avoid trapping cells within the viscous digest.

Any combination of tissue digestion enzymes can be used. Typical concentrations for digestion using trypsin include, 0.1% to about 2% trypsin, e.g,. about 0.25% trypsin. Proteases can be used in combination, that is, two or more proteases in the same digestion reaction, or can be used sequentially in order to liberate placental cells, e.g., placental stem cells and placental multipotent cells. For example, in one embodiment, a placenta, or part thereof, is digested first with an appropriate amount of collagenase I at about 1 to about 2 mg/ml for, e.g., 30 minutes, followed by digestion with trypsin, at a concentration of about 0.25%, for, e.g., 10 minutes, at 37°C. Serine proteases are preferably used consecutively following use of other enzymes.

In another embodiment, the tissue can further be disrupted by the addition of a chelator, e.g., ethylene glycol bis(2-aminoethyl ether)-N,N,N'N'-tetraacetic acid (EGTA) or ethylenediaminetetraacetic acid (EDTA) to the stem cell collection composition comprising the stem cells, or to a solution in which the tissue is disrupted and/or digested prior to isolation of the stem cells with the stem cell collection composition.

Following digestion, the digestate is washed, for example, three times with culture medium, and the washed cells are seeded into culture flasks. The cells are then isolated by differential adherence, and characterized for, e.g., viability, cell surface markers, differentiation, and the like.

It will be appreciated that where an entire placenta, or portion of a placenta comprising both fetal and maternal cells (for example, where the portion of the placenta comprises the chorion or cotyledons), the placental cells isolated can comprise a mix of placental cells derived from both fetal and maternal sources. Where a portion of the placenta that comprises no, or a negligible number of, maternal cells (for example, amnion), the placental cells isolated therefrom will comprise almost exclusively fetal placental cells (that is, placental cells having the genotype of the fetus).

Placental cells, *e*.*g*., the placental cells described in Section 5.1, above, can be isolated from disrupted placental tissue by differential trypsinization (*see* Section 5.2.5, below) followed by culture in one or more new culture containers in fresh proliferation medium, optionally followed by a second differential trypsinization step.

### 5.2.4 Placental Perfusion

Placental cells, *e.g.*, the placental cells described in Section 5.1, above, can also be obtained by perfusion of the mammalian placenta. Methods of perfusing mammalian placenta to obtain placental cells are disclosed, e.g., in Hariri, U.S. Patent Nos. 7,045,148 and 7,255,729, in U.S. Patent Application Publication Nos. 2007/0275362 and 2007/0190042.

Placental cells can be collected by perfusion, e.g., through the placental vasculature, using, e.g., a cell collection composition as a perfusion solution. In one embodiment, a mammalian placenta is perfused by passage of perfusion solution through either or both of the umbilical artery and umbilical vein. The flow of perfusion solution through the placenta may be accomplished using, e.g., gravity flow into the placenta. Preferably, the perfusion solution is forced through the placenta using a pump, e.g., a peristaltic pump. The umbilical vein can be, e.g., cannulated with a cannula, e.g., a TEFLON^{®} or plastic cannula, that is connected to a sterile connection apparatus, such as sterile tubing. The sterile connection apparatus is connected to a perfusion manifold.

In preparation for perfusion, the placenta is preferably oriented (e.g., suspended) in such a manner that the umbilical artery and umbilical vein are located at the highest point of the placenta. The placenta can be perfused by passage of a perfusion fluid through the placental vasculature and surrounding tissue. The placenta can also be perfused by passage of a perfusion fluid into the umbilical vein and collection from the umbilical arteries, or passage of a perfusion fluid into the umbilical arteries and collection from the umbilical vein.

In one embodiment, for example, the umbilical artery and the umbilical vein are connected simultaneously, e.g., to a pipette that is connected via a flexible connector to a reservoir of the perfusion solution. The perfusion solution is passed into the umbilical vein and artery. The perfusion solution exudes from and/or passes through the walls of the blood vessels into the surrounding tissues of the placenta, and is collected in a suitable open vessel from the surface of the placenta that was attached to the uterus of the mother during gestation. The perfusion solution may also be introduced through the umbilical cord opening and allowed to flow or percolate out of openings in the wall of the placenta which interfaced with the maternal uterine wall. Placental cells that are collected by this method, which can be referred to as a "pan" method, are typically a mixture of fetal and maternal cells.

In another embodiment, the perfusion solution is passed through the umbilical veins and collected from the umbilical artery, or is passed through the umbilical artery and collected from the umbilical veins. Placental cells collected by this method, which can be referred to as a "closed circuit" method, are typically almost exclusively fetal.

It will be appreciated that perfusion using the pan method, that is, whereby perfusate is collected after it has exuded from the maternal side of the placenta, results in a mix of fetal and maternal cells. As a result, the cells collected by this method can comprise a mixed population of placental cells, e.g., placental stem cells or placental multipotent cells, of both fetal and maternal origin. In contrast, perfusion solely through the placental vasculature in the closed circuit method, whereby perfusion fluid is passed through one or two placental vessels and is collected solely through the remaining vessel(s), results in the collection of a population of placental cells almost exclusively of fetal origin.

The closed circuit perfusion method can, in one embodiment, be performed as follows. A post-partum placenta is obtained within about 48 hours after birth. The umbilical cord is clamped and cut above the clamp. The umbilical cord can be discarded, or can processed to recover, e.g., umbilical cord stem cells, and/or to process the umbilical cord membrane for the production of a biomaterial. The amniotic membrane can be retained during perfusion, or can be separated from the chorion, e.g., using blunt dissection with the fingers. If the amniotic membrane is separated from the chorion prior to perfusion, it can be, e.g., discarded, or processed, e.g., to obtain stem cells by enzymatic digestion, or to produce, e.g., an amniotic membrane biomaterial, e.g., the biomaterial described in U.S. Application Publication No. 2004/0048796. After cleaning the placenta of all visible blood clots and residual blood, e.g., using sterile gauze, the umbilical cord vessels are exposed, e.g., by partially cutting the umbilical cord membrane to expose a cross-section of the cord. The vessels are identified, and opened, e.g., by advancing a closed alligator clamp through the cut end of each vessel. The apparatus, e.g., plastic tubing connected to a perfusion device or peristaltic pump, is then inserted into each of the placental arteries. The pump can be any pump suitable for the purpose, e.g., a peristaltic pump. Plastic tubing, connected to a sterile collection reservoir, e.g., a blood bag such as a 250 mL collection bag, is then inserted into the placental vein. Alternatively, the tubing connected to the pump is inserted into the placental vein, and tubes to a collection reservoir(s) are inserted into one or both of the placental arteries. The placenta is then perfused with a volume of perfusion solution, e.g., about 750 ml of perfusion solution. Cells in the perfusate are then collected, e.g., by centrifugation. In certain embodiments, the placenta is perfused with perfusion solution, e.g., 100-300 mL perfusion solution, to remove residual blood prior to perfusion to collect placental cells, e.g., placental stem cells and/or placental multipotent cells. In another embodiment, the placenta is not perfused with perfusion solution to remove residual blood prior to perfusion to collect placental cells.

In one embodiment, the proximal umbilical cord is clamped during perfusion, and more preferably, is clamped within 4-5 cm (centimeter) of the cord's insertion into the placental disc.

The first collection of perfusion fluid from a mammalian placenta during the exsanguination process is generally colored with residual red blood cells of the cord blood and/or placental blood. The perfusion fluid becomes more colorless as perfusion proceeds and the residual cord blood cells are washed out of the placenta. Generally from 30 to 100 ml (milliliter) of perfusion fluid is adequate to initially exsanguinate the placenta, but more or less perfusion fluid may be used depending on the observed results.

The volume of perfusion liquid used to isolate placental cells may vary depending upon the number of cells to be collected, the size of the placenta, the number of collections to be made from a single placenta, etc. In various embodiments, the volume of perfusion liquid may be from 50 mL to 5000 mL, 50 mL to 4000 mL, 50 mL to 3000 mL, 100 mL to 2000 mL, 250 mL to 2000 mL, 500 mL to 2000 mL, or 750 mL to 2000 mL. Typically, the placenta is perfused with 700-800 mL of perfusion liquid following exsanguination.

The placenta can be perfused a plurality of times over the course of several hours or several days. Where the placenta is to be perfused a plurality of times, it may be maintained or cultured under aseptic conditions in a container or other suitable vessel, and perfused with the cell collection composition, or a standard perfusion solution (e.g., a normal saline solution such as phosphate buffered saline ("PBS")) with or without an anticoagulant (e.g., heparin, warfarin sodium, coumarin, bishydroxycoumarin), and/or with or without an antimicrobial agent (e.g., β-mercaptoethanol (0.1 mM); antibiotics such as streptomycin (e.g., at 40-100 µg/ml), penicillin (e.g., at 40U/ml), amphotericin B (e.g., at 0.5 µg/ml). In one embodiment, an isolated placenta is maintained or cultured for a period of time without collecting the perfusate, such that the placenta is maintained or cultured for 1, 2, 3, 4, 5, 6, 7, 8, 9, 10, 11, 12, 13, 14, 15, 16, 17, 18, 19, 20, 21, 22, 23, or 24 hours, or 2 or 3 or more days before perfusion and collection of perfusate. The perfused placenta can be maintained for one or more additional time(s), e.g., 1, 2, 3, 4, 5, 6, 7, 8, 9, 10, 11, 12, 13, 14, 15, 16, 17, 18, 19, 20, 21, 22, 23, 24 or more hours, and perfused a second time with, e.g., 700-800 mL perfusion fluid. The placenta can be perfused 1, 2, 3, 4, 5 or more times, for example, once every 1, 2, 3, 4, 5 or 6 hours. In a preferred embodiment, perfusion of the placenta and collection of perfusion solution, e.g., cell collection composition, is repeated until the number of recovered nucleated cells falls below 100 cells/ml. The perfusates at different time points can be further processed individually to recover time-dependent populations of cells, e.g., stem cells. Perfusates from different time points can also be pooled. In a preferred embodiment, placental cells are collected at a time or times between about 8 hours and about 18 hours post-expulsion.

Perfusion preferably results in the collection of significantly more placental cells than the number obtainable from a mammalian placenta not perfused with said solution, and not otherwise treated to obtain placental cells (e.g., by tissue disruption, e.g., enzymatic digestion). In this context, "significantly more" means at least 10% more. Perfusion yields significantly more placental cells than, e.g., the number of placental cells isolatable from culture medium in which a placenta, or portion thereof, has been cultured.

Placental cells can be isolated from placenta by perfusion with a solution comprising one or more proteases or other tissue-disruptive enzymes. In a specific embodiment, a placenta or portion thereof (e.g., amniotic membrane, amnion and chorion, placental lobule or cotyledon, umbilical cord, or combination of any of the foregoing) is brought to 25-37°C, and is incubated with one or more tissue-disruptive enzymes in 200 mL of a culture medium for 30 minutes. Cells from the perfusate are collected, brought to 4°C, and washed with a cold inhibitor mix comprising 5 mM EDTA, 2 mM dithiothreitol and 2 mM beta-mercaptoethanol. The placental cells are washed after several minutes with a cold (*e.g.,* 4°C) stem cell collection composition.

### 5.2.5 Isolation, Sorting, and Characterization of Placental Cells

The isolated placental cells, *e*.*g*., the cells described in Section 5.1, above, whether obtained by perfusion or physical disruption, *e*.*g*., by enzymatic digestion, can initially be purified from (*i.e.*, be isolated from) other cells by Ficoll gradient centrifugation. Such centrifugation can follow any standard protocol for centrifugation speed, etc. In one embodiment, for example, cells collected from the placenta are recovered from perfusate by centrifugation at 5000 × g for 15 minutes at room temperature, which separates cells from, e.g., contaminating debris and platelets. In another embodiment, placental perfusate is concentrated to about 200 ml, gently layered over Ficoll, and centrifuged at about 1100 × g for 20 minutes at 22°C, and the low-density interface layer of cells is collected for further processing.

Cell pellets can be resuspended in fresh stem cell collection composition, or a medium suitable for cell maintenance, e.g., stem cell maintenance, for example, IMDM serum-free medium containing 2U/ml heparin and 2 mM EDTA (GibcoBRL, NY). The total mononuclear cell fraction can be isolated, e.g., using Lymphoprep (Nycomed Pharma, Oslo, Norway) according to the manufacturer's recommended procedure.

Placental cells obtained by perfusion or digestion can, for example, be further, or initially, isolated by differential trypsinization using, e.g., a solution of 0.05% trypsin with 0.2% EDTA (Sigma, St. Louis MO). Differential trypsinization is possible because the isolated placental cells, which are tissue culture plastic-adherent, typically detach from the plastic surfaces within about five minutes whereas other adherent populations typically require more than 20-30 minutes incubation. The detached placental cells can be harvested following trypsinization and trypsin neutralization, using, e.g., Trypsin Neutralizing Solution (TNS, Cambrex). In one embodiment of isolation of adherent cells, aliquots of, for example, about 5-10 × 10⁶ cells are placed in each of several T-75 flasks, preferably fibronectin-coated T75 flasks. In such an embodiment, the cells can be cultured with commercially available Mesenchymal Stem Cell Growth Medium (MSCGM) (Cambrex), and placed in a tissue culture incubator (37°C, 5% CO2). After 10 to 15 days, non-adherent cells are removed from the flasks by washing with PBS. The PBS is then replaced by MSCGM. Flasks are preferably examined daily for the presence of various adherent cell types and in particular, for identification and expansion of clusters of fibroblastoid cells.

The number and type of cells collected from a mammalian placenta can be monitored, for example, by measuring changes in morphology and cell surface markers using standard cell detection techniques such as flow cytometry, cell sorting, immunocytochemistry (e.g., staining with tissue specific or cell-marker specific antibodies) fluorescence activated cell sorting (FACS), magnetic activated cell sorting (MACS), by examination of the morphology of cells using light or confocal microscopy, and/or by measuring changes in gene expression using techniques well known in the art, such as PCR and gene expression profiling. These techniques can be used, too, to identify cells that are positive for one or more particular markers. For example, using antibodies to CD34, one can determine, using the techniques above, whether a cell comprises a detectable amount of CD34; if so, the cell is CD34+. Likewise, if a cell produces enough OCT-4 RNA to be detectable by RT-PCR, or significantly more OCT-4 RNA than an adult cell, the cell is OCT-4+. Antibodies to cell surface markers (e.g., CD markers such as CD34) and the sequence of stem cell-specific genes, such as OCT-4, are well-known in the art.

Placental cells, particularly cells that have been isolated by Ficoll separation, differential adherence, or a combination of both, may be sorted using a fluorescence activated cell sorter (FACS). Fluorescence activated cell sorting (FACS) is a well-known method for separating particles, including cells, based on the fluorescent properties of the particles (Kamarch, 1987, Methods Enzymol, 151:150-165). Laser excitation of fluorescent moieties in the individual particles results in a small electrical charge allowing electromagnetic separation of positive and negative particles from a mixture. In one embodiment, cell surface marker-specific antibodies or ligands are labeled with distinct fluorescent labels. Cells are processed through the cell sorter, allowing separation of cells based on their ability to bind to the antibodies used. FACS sorted particles may be directly deposited into individual wells of 96-well or 384-well plates to facilitate separation and cloning.

In one sorting scheme, cells from placenta, e.g., PDACs are sorted on the basis of expression of one or more of the markers CD34, CD38, CD44, CD45, CD73, CD105, OCT-4 and/or HLA-G. This can be accomplished in connection with procedures to select such cells on the basis of their adherence properties in culture. For example, tissue culture plastic adherence selection can be accomplished before or after sorting on the basis of marker expression. In one embodiment, for example, cells are sorted first on the basis of their expression of CD34; CD34-cells are retained, and CD34- cells that are additionally CD200+ and HLA-G- are separated from all other CD34- cells. In another embodiment, cells from placenta are sorted based on their expression of markers CD200 and/or HLA-G; for example, cells displaying CD200 and lacking HLA-G are isolated for further use. Cells that express, e.g., CD200 and/or lack, e.g., HLA-G can, in a specific embodiment, be further sorted based on their expression of CD73 and/or CD105, or epitopes recognized by antibodies SH2, SH3 or SH4, or lack of expression of CD34, CD38 or CD45. For example, in another embodiment, placental cells are sorted by expression, or lack thereof, of CD200, HLA-G, CD73, CD105, CD34, CD38 and CD45, and placental cells that are CD200+, HLA-G-, CD73+, CD105+, CD34-, CD38- and CD45- are isolated from other placental cells for further use.

In specific embodiments of any of the above embodiments of sorted placental cells, at least 50%, 60%, 70%, 80%, 90% or 95% of the cells in a cell population remaining after sorting are said isolated placental cells. Placental cells can be sorted by one or more of any of the markers described in Section 5.1, above.

In a specific embodiment, for example, placental cells that are (1) adherent to tissue culture plastic, and (2) CD10+, CD34- and CD105+ are sorted from (i.e., isolated from) other placental cells. In another specific embodiment, placental cells that are (1) adherent to tissue culture plastic, and (2) CD10+, CD34-, CD105+ and CD200+ are sorted from (i.e., isolated from) other placental cells. In another specific embodiment, placental cells that are (1) adherent to tissue culture plastic, and (2) CD10+, CD34-, CD45-, CD90+, CD105+ and CD200+ are sorted from (i.e., isolated from) other placental cells.

With respect to nucleotide sequence-based detection of placental cells, sequences for the markers listed herein are readily available in publicly-available databases such as GenBank or EMBL.

With respect to antibody-mediated detection and sorting of placental cells, e.g., placental stem cells or placental multipotent cells, any antibody, specific for a particular marker, can be used, in combination with any fluorophore or other label suitable for the detection and sorting of cells (e.g., fluorescence-activated cell sorting). Antibody/fluorophore combinations to specific markers include, but are not limited to, fluorescein isothiocyanate (FITC) conjugated monoclonal antibodies against HLA-G (available from Serotec, Raleigh, North Carolina), CD10 (available from BD Immunocytometry Systems, San Jose, California), CD44 (available from BD Biosciences Pharmingen, San Jose, California), and CD105 (available from R&D Systems Inc., Minneapolis, Minnesota); phycoerythrin (PE) conjugated monoclonal antibodies against CD44, CD200, CD117, and CD13 (BD Biosciences Pharmingen); phycoerythrin-Cy7 (PE Cy7) conjugated monoclonal antibodies against CD33 and CD10 (BD Biosciences Pharmingen); allophycocyanin (APC) conjugated streptavidin and monoclonal antibodies against CD38 (BD Biosciences Pharmingen); and Biotinylated CD90 (BD Biosciences Pharmingen). Other antibodies that can be used include, but are not limited to, CD133-APC (Miltenyi), KDR-Biotin (CD309, Abcam), CytokeratinK-Fitc (Sigma or Dako), HLA ABC-Fitc (BD), HLA DR,DQ,DP-PE (BD), β-2-microglobulin-PE (BD), CD80-PE (BD) and CD86-APC (BD). Other antibody/label combinations that can be used include, but are not limited to, CD45-PerCP (peridin chlorophyll protein); CD44-PE; CD19-PE; CD10-F (fluorescein); HLA-G-F and 7-amino-actinomycin-D (7-AAD); HLA-ABC-F; and the like. This list is not exhaustive, and other antibodies from other suppliers are also commercially available.

The isolated placental cells provided herein can be assayed for CD117 or CD133 using, for example, phycoerythrin-Cy5 (PE Cy5) conjugated streptavidin and biotin conjugated monoclonal antibodies against CD117 or CD133; however, using this system, the cells can appear to be positive for CD117 or CD133, respectively, because of a relatively high background.

The isolated placental cells can be labeled with an antibody to a single marker and detected and/sorted. Placental cells can also be simultaneously labeled with multiple antibodies to different markers.

In another embodiment, magnetic beads can be used to separate cells. The cells may be sorted using a magnetic activated cell sorting (MACS) technique, a method for separating particles based on their ability to bind magnetic beads (0.5-100 µm diameter). A variety of useful modifications can be performed on the magnetic microspheres, including covalent addition of antibody that specifically recognizes a particular cell surface molecule or hapten. The beads are then mixed with the cells to allow binding. Cells are then passed through a magnetic field to separate out cells having the specific cell surface marker. In one embodiment, these cells can then isolated and re-mixed with magnetic beads coupled to an antibody against additional cell surface markers. The cells are again passed through a magnetic field, isolating cells that bound both the antibodies. Such cells can then be diluted into separate dishes, such as microtiter dishes for clonal isolation.

Isolated placental cells can also be characterized and/or sorted based on cell morphology and growth characteristics. For example, isolated placental cells can be characterized as having, and/or selected on the basis of, e.g., a fibroblastoid appearance in culture. The isolated placental cells can also be characterized as having, and/or be selected, on the basis of their ability to form embryoid-like bodies. In one embodiment, for example, placental cells that are fibroblastoid in shape, express CD73 and CD105, and produce one or more embryoid-like bodies in culture are isolated from other placental cells. In another embodiment, OCT-4+ placental cells that produce one or more embryoid-like bodies in culture are isolated from other placental cells.

In another embodiment, isolated placental cells can be identified and characterized by a colony forming unit assay. Colony forming unit assays are commonly known in the art, such as MesenCult^{™} medium (Stem Cell Technologies, Inc., Vancouver British Columbia).

The isolated placental cells can be assessed for viability, proliferation potential, and longevity using standard techniques known in the art, such as trypan blue exclusion assay, fluorescein diacetate uptake assay, propidium iodide uptake assay (to assess viability); and thymidine uptake assay, MTT (3-(4,5-Dimethylthiazol-2-yl)-2,5-diphenyltetrazolium bromide) cell proliferation assay (to assess proliferation). Longevity may be determined by methods well known in the art, such as by determining the maximum number of population doubling in an extended culture.

Isolated placental cells, e.g., the isolated placental cells described in Section 5.1, above, can also be separated from other placental cells using other techniques known in the art, e.g., selective growth of desired cells (positive selection), selective destruction of unwanted cells (negative selection); separation based upon differential cell agglutinability in the mixed population as, for example, with soybean agglutinin; freeze-thaw procedures; filtration; conventional and zonal centrifugation; centrifugal elutriation (counter-streaming centrifugation); unit gravity separation; countercurrent distribution; electrophoresis; and the like.

### 5.2.6 Crvooreserved Isolated Placental Cells

In certain embodiments, the isolated placental cell populations useful in the methods described herein have been= preserved, for example, cryopreserved, prior to use in the methods described herien. Methods for cryopreservation of cells, such as stem cells, are well known in the art. In certain embodiments, the placental cells are prepared in a form that is easily administrable to an individual, e.g., an isolated placental cell population that is contained within a container that is suitable for medical use. Such a container can be, for example, a syringe, sterile plastic bag, flask, jar, or other container from which the isolated placental cell population can be easily dispensed. For example, the container can be a blood bag or other plastic, medically-acceptable bag suitable for the intravenous administration of a liquid to a recipient. The container, in certain embodiments, is one that allows for cryopreservation of the combined cell population.

The cryopreserved isolated placental cell population can comprise isolated placental cell derived from a single donor, or from multiple donors. The isolated placental cell population can be completely HLA-matched to an intended recipient, or partially or completely HLA-mismatched.

Thus, in one embodiment, isolated placental cells can be used in the methods and described herein in the form of a composition comprising a tissue culture plastic-adherent placental cell population in a container. In a specific embodiment, the isolated placental cells are cryopreserved. In another specific embodiment, the container is a bag, flask, or jar. In another specific embodiment, the composition comprises one or more compounds that facilitate cryopreservation of the combined cell population. In another specific embodiment, said isolated placental cell population is contained within a physiologically-acceptable aqueous solution. In another specific embodiment, said physiologically-acceptable aqueous solution is a 0.9% NaCl solution. In another specific embodiment, said isolated placental cell population comprises placental cells that are HLA-matched to a recipient of said cell population. In another specific embodiment, said combined cell population comprises placental cells that are at least partially HLA-mismatched to a recipient of said cell population. In another specific embodiment, said isolated placental cells are derived from a plurality of donors.

In certain embodiments, the isolated placental cells in the container are isolated CD10+, CD34-, CD105+ placental cells, wherein said cells have been cryopreserved, and are contained within a container. In a specific embodiment, said CD10+, CD34-, CD105+ placental cells are also CD200+. In another specific embodiment, said CD10+, CD34-, CD105+, CD200+ placental cells are also CD45- or CD90+. In another specific embodiment, said CD10+, CD34-, CD105+, CD200+ placental cells are also CD45- and CD90+. In another specific embodiment, the CD34-, CD10+, CD105+ placental cells are additionally one or more of CD13+, CD29+, CD33+, CD38-, CD44+, CD45-, CD54+, CD62E-, CD62L-, CD62P-, SH3+ (CD73+), SH4+ (CD73+), CD80-, CD8Cr, CD90+, SH2+ (CD105+), CD106/VCAM+, CD117-, CD144/VE-cadherindim, CD184/CXCR4-, CD200+, CD133-, OCT-4+, SSEA3-, SSEA4-, ABC-p+, KDR- (VEGFR2-), HLA-A,B,C+, HLA-DP,DQ,DR-, HLA-G-, or Programmed Death-1 Ligand (PDL1)+, or any combination thereof. In another specific embodiment, the CD34-, CD10+, CD105+ placental cells are additionally CD13+, CD29+, CD33+, CD38-, CD44+, CD45-, CD54/ICAM+, CD62E-, CD62L-, CD62P-, SH3+ (CD73+), SH4+ (CD73+), CD80-, CD86-, CD90+, SH2+ (CD105+), CD106/VCAM+, CD117-, CD144/VE-cadherindim, CD184/CXCR4-, CD200+, CD133-, OCT-4+, SSEA3-, SSEA4-, ABC-p+, KDR- (VEGFR2-), HLA-A,B,C+, HLA-DP,DQ,DR-, HLA-G-, and Programmed Death-1 Ligand (PDL1)+.

In certain other embodiments, the above-referenced isolated placental cells are isolated CD200+, HLA-G- placental cells, wherein said cells have been cryopreserved, and are contained within a container. In another embodiment, the isolated placental cells are CD73+, CD105+, CD200+ cells that have been cryopreserved, and are contained within a container. In another embodiment, the isolated placental cells are CD200+, OCT-4+ stem cells that have been cryopreserved, and are contained within a container. In another embodiment, the isolated placental cells are CD73+, CD105+ cells that have been cryopreserved, and are contained within a container, and wherein said isolated placental cells facilitate the formation of one or more embryoid-like bodies when cultured with a population of placental cells under conditions that allow for the formation of embryoid-like bodies. In another embodiment, the isolated placental cells are CD73+, CD105+, HLA-G- cells that have been cryopreserved, and are contained within a container. In another embodiment, the isolated placental cells are OCT-4+ placental cells that have been cryopreserved, and are contained within a container, and wherein said cells facilitate the formation of one or more embryoid-like bodies when cultured with a population of placental cells under conditions that allow for the formation of embryoid-like bodies.

In another specific embodiment, the above-referenced isolated placental cells are placental stem cells or placental multipotent cells that are CD34-, CD10+ and CD105+ as detected by flow cytometry (e.g., PDACs). In another specific embodiment, the isolated CD34-, CD10+, CD105+ placental cells have the potential to differentiate into cells of a neural phenotype, cells of an osteogenic phenotype, or cells of a chondrogenic phenotype. In another specific embodiment, the isolated CD34-, CD10+, CD105+ placental cells are additionally CD200+. In another specific embodiment, the isolated CD34-, CD10+, CD105+ placental cells are additionally CD90+ or CD45-, as detected by flow cytometry. In another specific embodiment, the isolated CD34-, CD10+, CD105+ placental cells are additionally CD90+ or CD45-, as detected by flow cytometry. In another specific embodiment, the CD34-, CD10+, CD105+, CD200+ placental cells are additionally CD90+ or CD45-, as detected by flow cytometry. In another specific embodiment, the CD34-, CD10+, CD105+, CD200+ cells are additionally CD90+ and CD45-, as detected by flow cytometry. In another specific embodiment, the CD34-, CD10+, CD105+, CD200+, CD90+, CD45- cells are additionally CDSO- and CD86-, as detected by flow cytometry. In another specific embodiment, the CD34-, CD10+, CD105+ cells are additionally one or more of CD29+, CD38-, CD44+, CD54+, CD80-, CD86-, SH3+ or SH4+. In another specific embodiment, the cells are additionally CD44+. In a specific embodiment of any of the isolated CD34-, CD10+, CD105+ placental cells above, the cells are additionally one or more of CD117-, CD133-, KDR- (VEGFR2-), HLA-A,B,C+, HLA-DP,DQ,DR-, and/or PDL1+.

In a specific embodiment of any of the foregoing cryopreserved isolated placental cells, said container is a bag or tube. In various specific embodiments, said container comprises about, at least, or at most 1 × 10⁶ said isolated placental cells, 5 × 10⁶ said isolated placental cells, 1 × 10⁷ said isolated placental cells, 5 × 10⁷ said isolated placental cells, 1 × 10⁸ said isolated placental cells, 5 × 10⁸ said isolated placental cells, 1 × 10⁹ said isolated placental cells, 5 × 10⁹ said isolated placental cells, 1 × 10¹⁰ said isolated placental cells, or 1 × 10¹⁰ said isolated placental cells. In other specific embodiments of any of the foregoing cryopreserved populations, said isolated placental cells have been passaged about, at least, or no more than 5 times, no more than 10 times, no more than 15 times, or no more than 20 times. In another specific embodiment of any of the foregoing cryopreserved isolated placental cells, said isolated placental cells have been expanded within said container.

In certain embodiments, a single unit dose of placental derived adherent cells used in the methods described herein can comprise, in various embodiments, about, at least, or no more than 1 × 10³, 3 × 10³, 5 × 10³, 1 × 10⁴, 3 × 10⁴, 5 × 10⁴, 1 × 10⁵, 3 × 10⁵, 5 × 10⁵, 1 × 10⁶, 3 × 10⁶ , 5 × 10⁶, 1 × 10⁷, 3 × 10⁷, 5 × 10⁷, 1 × 10⁸, 3 × 10⁸, 5 × 10⁸, 1 × 10⁹, 5 × 10⁹, or 1 × 10¹⁰ placental cells. In certain embodiments, a single unit dose of placental derived adherent cells can comprise between 1 × 10³ to 3 × 10³, 3 × 10³ to 5 × 10³, 5 × 10³ to 1 × 10⁴, 1 × 10⁴ to 3 × 10⁴, 3 × 10⁴ to 5 × 10⁴, 5 × 10⁴ to 1 × 10⁵, 1 × 10⁵ to 3 × 10⁵, 3 × 10⁵ to 5 × 10⁵, 5 × 10⁵ to 1 × 10⁶, 1 × 10⁶ to 3 × 10⁶, 3 × 10⁶ to 5 × 10⁶, 5 × 10⁶ to 1 × 10⁷, 1 × 10⁷ to 3 × 10⁷, 3x 10⁷ to 5 × 10⁷, 5 × 10⁷ to 1 × 10⁸, 1 × 10⁸ to 3 × 10⁸, 3 × 10⁸ to 5 × 10⁸, 5 × 10⁸ to 1 × 10⁹, 1 × 10⁹ to 5 × 10⁹, or 5 × 10⁹ to 1 × 10¹⁰ placental cells.

### 6. EXAMPLES

### 6.1 EXAMPLE 1: CELL POTENCY ASSAY

This example describes a cell potency assay performed using CD10+, CD34-, CD105+, CD200+ placental stem cells.

Three distinct lots of cryopreserved CD10+, CD34-, CD105+, CD200+ placental stem cells were used in the experiment. From each lot, three separate cell preparations were obtained. Four aliquots from each cell preparation were seeded in a 48-well plate at 10,000 cells/cm² (9200 total cells per well).

The cells then were cultured for 24 hours or 48 hours ("recovery cultures") in the same standard cell culture media (basal DMEM). Percent confluence of the cells during the course of the recovery culture period was assessed visually on an hourly basis using the IncuCyte ZOOM^{®} In-Incubator Imaging and Image Analysis System. After the 24-hour or 48-hour recovery period, the cells were cultured in medium comprising interleukin-1 beta in order to induce PGE2 production, and cultured for an additional 24 hours. Following the 24-hour induction culture, conditioned media was collected from the cell cultures, and the amount of PGE2 in the conditioned media was assessed by ELISA.

As shown in Figure 1, use of a 48 hour recovery culture period, as opposed to a 24-hour recovery culture period, improved inter-assay as well as intra-assay variability, as measured by percent confluence of the cells. The coefficient of variance (%CV) for the cell preparations cultured for 48 hours was less than the coefficient of variance for the cell preparations cultured for 24 hours in each instance. The same result was observed when the experiment was repeated. See Figure 2.

As shown in Figure 3, a cell potency assay utilizing a 48-hour recovery culture is superior to a cell potency assay that uses a 24-hour recovery culture. Improvement in assay variability was determined by measurement of PGE2, a surrogate factor for cell potency of CD10+, CD34-, CD105+, CD200+ placental stem cells. In particular, PGE2 expression acts as a surrogate for immunosuppressive activity of the placental stem cells. In each of three separate lots of placental stem cells tested, the coefficient of variance (%CV) in PGE2 levels measured using cells from a 48-hour recovery culture was less than the coefficient of variance in PGE2 levels measured using cells from a 24-hour recovery culture.

To further confirm superiority of the cell potency assay that utilizes a 48-hour recovery culture, a large scale experiment, using numerous cell preparations was performed. See Figure 4. It again was demonstrated that increasing recovery culture time to 48 hours reduced intra-assay variability (Figure 4A) and and reduced inter-assay variability (Figure 4B) as compared to a cell potency assay that utilized a 24-hour recovery culture. Further, it was demonstrated that increasing recovery culture length to 48 hours did not affect dynamic range (Figure 4C), but that the cell poteny assay utilizing a 48-hour recovery time had increased statistical power (Figure 4D).

In conclusion, this Example demonstrates that the recovery culture period employed in cell potency assays is a critical parameter.

### Equivalents:

The present disclosure is not to be limited in scope by the specific embodiments described herein. Indeed, various modifications of the subject matter provided herein, in addition to those described, will become apparent to those skilled in the art from the foregoing description.

## Claims

1. A method for assessing cell potency, said method comprising:
(a) the following steps (i) culturing a population of cells in a recovery culture, wherein the cell culture media is DMEM, and wherein the cells are placental cells, for about 48 hours; (ii) further culturing the cells in an induction culture, wherein the cells are cultured in a medium that comprises one or more agents capable of inducing production of one or more genes in the cells; and (iii) performing an assay to determine whether the cells produce a marker that acts as a surrogate for potency of the cells, or
(b) the following steps: (i) culturing a population of cells in a recovery culture, wherein the cell culture media is DMEM, and wherein the cells are placental cells, for about 48 hours; (ii) further culturing the cells in an induction culture, wherein the cells are cultured in a medium that comprises an agent capable of inducing production of prostaglandin E2 (PGE2) by the cells; and (iii) performing an assay to determine whether the cells produce PGE2, wherein expression of PGE2 is a surrogate for potency of the cells.

2. The method of claim 1(b), wherein said agent capable of inducing production of PGE2 by the cells is interleukin-1 (IL-1) beta.

3. The method of any one of claims 1-2, wherein the population of cells is suitable for use as a cell therapy.

4. The method of any one of claims 1-3, wherein:
(a) the cells are human cells, or
(b) the population of cells comprises placental stem cells, optionally wherein said placental cells adhere to tissue culture plastic and are CD34⁻, CD10⁺, CD105⁺ and CD200⁺, as detectable by flow cytometry.

5. The method of any one of claims 1-4, wherein the population of cells is obtained from a lot of cells that had been previously cryopreserved.

6. The method of any one of claims 1-5, wherein the marker that acts as a surrogate for potency of the cells in the population of cells correlates with immunosuppressive activity of the cells.

7. The method of any one of claims 1 (a), or 3-6 wherein the marker that acts as a surrogate for potency of the cells in the population of cells is PGE2.

8. The method of any one of claims 1(a), 3-6 wherein the marker that acts as a surrogate for potency of the cells in the population of cells is ANG, EGF, ENA-78, FGF2, Follistatin, G-CSF, GRO, HGF, IL-6, IL-8, Leptin, MCP-1, MCP-3, PDGFB, PLGF, Rantes, TGFB1, Thrombopoietin, TIMP1, TIMP2, uPAR, VEGF, VEGFD, angiopoietin-1, angiopoietin-2, PECAM-1 (CD31; platelet endothelial cell adhesion molecule), laminin and/or fibronectin.

9. The method of any one of claims 1-8, wherein said marker that acts as a surrogate for cell potency is detected by: ELISA, or a MulitplexBead Assay, or an assay that measures gene expression, optionally wherein said assay that measures gene expression is RT-PCR.

10. The method of any one of claims 1-9, further comprising collection of the conditioned media from the cell culture.

11. The method of any one of claims 1-10, wherein if the cells produce a marker that acts as a surrogate for potency of the cells, the original non-cultured population of cells is divided into a lot of cells suitable for administration to human subjects, optionally wherein said lot is cryopreserved.

12. The method of any one of claims 1-10, wherein the population of cells is from a lot of cells, optionally wherein said lot of cells is cryopreserved.

## Patentansprüche

1. Verfahren zur Beurteilung von Zellpotenz, wobei das Verfahren Folgendes umfasst:
(a) die folgenden Schritte: (i) das Kultivieren einer Population von Zellen in einer Gewinnungskultur, wobei das Zellkulturmedium DMEM ist und wobei die Zellen Plazentazellen sind, für etwa 48 Stunden; (ii) das weitere Kultivieren der Zellen in einer Induktionskultur, wobei die Zellen in einem Medium kultiviert werden, das ein oder mehrere Mittel umfasst, die in der Lage sind, die Produktion eines oder mehrerer Gene in den Zellen zu induzieren; und (iii) das Durchführen eines Tests, um zu bestimmen, ob die Zellen einen Marker produzieren, der als stellvertrend für die Potenz der Zellen gilt; oder
(b) die folgenden Schritte: (i) das Kultivieren einer Population von Zellen in einer Gewinnungskultur, wobei das Zellkulturmedium DMEM ist und wobei die Zellen Plazentazellen sind, für etwa 48 Stunden; (ii) das weitere Kultivieren der Zellen in einer Induktionskultur, wobei die Zellen in einem Medium kultiviert werden, das ein oder mehrere Mittel umfasst die in der Lage sind, die Produktion von Prostaglandin E2 (PGE2) durch die Zellen zu induzieren; und (iii) das Durchführen eines Tests, um zu bestimmen, ob die Zellen PGE2 produzieren, wobei die Expression von PGE2 ein stellvertretend für die Potenz der Zellen gilt.

2. Verfahren nach Anspruch 1(b), wobei das Mittel, das in der Lage ist, die Produktion von PGE2 durch die Zellen zu induzieren, Interleukin-1β (IL-1β) ist.

3. Verfahren nach einem der Ansprüche 1 bis 2, wobei die Population von Zellen zur Verwendung als Zelltherapie geeignet ist.

4. Verfahren nach einem der Ansprüche 1 bis 3, wobei:
(a) die Zellen menschliche Zellen sind oder
(b) die Population von Zellen Plazenta-Stammzellen umfasst, wobei die Plazentazellen gegebenenfalls an Gewebekultur-Kunststoff haften und CD34⁻, CD10⁺, CD105⁺ und CD200⁺ sind, was mittels Durchflusszytometrie detektierbar ist.

5. Verfahren nach einem der Ansprüche 1 bis 4, wobei die Population von Zellen aus einer Charge von Zellen erhalten wurde, die zuvor kryokonserviert worden war.

6. Verfahren nach einem der Ansprüche 1 bis 5, wobei der Marker, der als stellvertretend für die Potenz der Zellen in der Population von Zellen gilt, mit der immunsuppressiven Aktivität der Zellen korreliert.

7. Verfahren nach einem der Ansprüche 1(a) oder 3 bis 6, wobei der Marker, der als stellvertretend für die Potenz der Zellen in der Population von Zellen gilt, PGE2 ist.

8. Verfahren nach einem der Ansprüche 1(a), 3 bis 6, wobei der Marker, der als stellvertretend für die Potenz der Zellen in der Population von Zellen gilt, ANG, EGF, ENA-78, FGF2, Follistatin, G-CSF, GRO, HGF, IL-6, IL-8, Leptin, MCP-1, MCP- 3, PDGFB, PLGF, Rantes, TGFB 1, Thrombopoetin, TIMP1, TIMP2, uPAR, VEGF, VEGFD, Angiopoetin-1, Angiopoetin-2, PECAM- 1 (CD31; Thrombozyten-Endothelzellen-Adhäsionsmolekül), Laminin und/oder Fibronectin ist.

9. Verfahren nach einem der Ansprüche 1 bis 8, wobei der Marker, der als stellvertretend für die Zellpotenz gilt, durch Folgendes detektiert wird: ELISA, einen MultiplexBead-Assay oder einen Test, der die Genexpression misst, wobei dieser Test, der die Genexpression misst, gegebenenfalls RT-PCR ist.

10. Verfahren nach einem der Ansprüche 1 bis 9, das weiters das Sammeln des konditionierten Mediums aus der Zellkultur umfasst.

11. Verfahren nach einem der Ansprüche 1 bis 10, wobei, wenn die Zellen einen Marker produzieren, der als stellvertretend für die Potenz der Zellen gilt, die ursprüngliche, nicht kultivierte Population von Zellen in eine Charge von Zellen geteilt wird, die zur Verabreichung an menschliche Individuen geeignet ist, wobei die Charge gegebenenfalls kryokonserviert wird.

12. Verfahren nach einem der Ansprüche 1 bis 10, wobei die Population von Zellen von einer Charge von Zellen stammt, wobei diese Charge von Zellen gegebenenfalls kryokonserviert wird.

## Revendications

1. Procédé d'évaluation d'une puissance cellulaire, ledit procédé comprenant :
(a) les étapes suivantes consistant à (i) cultiver une population de cellules dans une culture de récupération, dans lequel le milieu de culture cellulaire est DMEM, et dans lequel les cellules sont des cellules placentaires, pendant environ 48 heures ; (ii) cultiver en outre les cellules dans une culture d'induction, dans lequel les cellules sont cultivées dans un milieu qui comprend un ou plusieurs agents capables d'induire la production d'un ou plusieurs gènes dans les cellules ; et (iii) effectuer un essai pour déterminer si les cellules produisent un marqueur qui agit comme un substitut à la puissance des cellules, ou
(b) les étapes suivantes consistant à : (i) cultiver une population de cellules dans une culture de récupération, dans lequel le milieu de culture cellulaire est DMEM, et dans lequel les cellules sont des cellules placentaires, pendant environ 48 heures ; (ii) cultiver en outre les cellules dans une culture d'induction, dans lequel les cellules sont cultivées dans un milieu qui comprend un agent capable d'induire la production de prostaglandine E2 (PGE2) par les cellules ; et (iii) effectuer un essai pour déterminer si les cellules produisent PGE2, dans lequel l'expression de PGE2 est un substitut à la puissance des cellules.

2. Procédé selon la revendication 1(b), dans lequel ledit agent capable d'induire la production de PGE2 par les cellules est l'interleukine-1 (IL-1) bêta.

3. Procédé selon l'une quelconque des revendications 1 et 2, dans lequel la population de cellules est adaptée à une utilisation comme thérapie cellulaire.

4. Procédé selon l'une quelconque des revendications 1 à 3, dans lequel :
(a) les cellules sont des cellules humaines, ou
(b) la population de cellules comprend des cellules souches placentaires, facultativement dans lequel lesdites cellules placentaires adhèrent à du plastique pour culture tissulaire et sont CD34-, CD10⁺, CD105⁺ et CD200⁺, comme détectable par cytométrie en flux.

5. Procédé selon l'une quelconque des revendications 1 à 4, dans lequel la population de cellules est obtenue à partir d'un lot de cellules qui ont été préalablement cryoconservées.

6. Procédé selon l'une quelconque des revendications 1 à 5, dans lequel le marqueur qui agit comme un substitut à la puissance des cellules dans la population de cellules est corrélé à une activité immunosuppressive des cellules.

7. Procédé selon l'une quelconque des revendications 1 (a), ou 3 à 6, dans lequel le marqueur qui agit comme un substitut à la puissance des cellules dans la population de cellules est PGE2.

8. Procédé selon l'une quelconque des revendications 1 (a), 3 à 6, dans lequel le marqueur qui agit comme un substitut à la puissance des cellules dans la population de cellules est ANG, EGF, ENA-78, FGF2, Follistatine, G-CSF, GRO, HGF, IL-6, IL-8, Leptine, MCP-1, MCP-3, PDGFB, PLGF, Rantes, TGFB1, Thrombopoïétine, TIMP1, TIMP2, uPAR, VEGF, VEGFD, angiopoïétine-1, angiopoïétine-2, PECAM-1 (CD31 ; molécule d'adhésion de cellules endothéliales-plaquettes), laminine et/ou fibronectine.

9. Procédé selon l'une quelconque des revendications 1 à 8, dans lequel ledit marqueur qui agit comme un substitut à la puissance cellulaire est détecté par : essai ELISA, ou un essai MulitplexBead, ou un essai qui mesure une expression génique, facultativement dans lequel ledit essai qui mesure une expression génique est RT-PCR.

10. Procédé selon l'une quelconque des revendications 1 à 9, comprenant en outre la collecte du milieu conditionné provenant de la culture cellulaire.

11. Procédé selon l'une quelconque des revendications 1 à 10, dans lequel, si les cellules produisent un marqueur qui agit en tant que substitut à la puissance des cellules, la population de cellules originale non cultivée est divisée en un lot de cellules appropriées à une administration à des sujets humains, dans lequel ledit lot est facultativement cryoconservé.

12. Procédé selon l'une quelconque des revendications 1 à 10, dans lequel la population de cellules provient d'un lot de cellules, facultativement dans lequel ledit lot de cellules est cryoconservé.
